(19)

Europäisches
Patentamt
European
Patent Office
Office européen
des brevets

(11) **EP 4 356 916 A1**

(12) **EUROPEAN PATENT APPLICATION**
published in accordance with Art. 153(4) EPC

(43) Date of publication:
**24.04.2024 Bulletin 2024/17**

(21) Application number: **23833282.9**

(22) Date of filing: **03.05.2023**

(51) International Patent Classification (IPC):
***A61K 35/28*** (2015.01)

(86) International application number:
**PCT/KR2023/006017**

(87) International publication number:
**WO 2024/048899 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.08.2022 KR 20220109086
23.02.2023 KR 20230024574**

(71) Applicants:
• **Seoul National University R&DB Foundation
Gwanak-gu
Seoul 08826 (KR)**
• **Industry-University Cooperation Foundation
Hanyang University
Seoul 04763 (KR)**

(72) Inventors:
• **CHANG, Mi Sook
Seoul 03080 (KR)**
• **KOH, Seong Ho
Seoul 04763 (KR)**
• **LEE, Eun Ji
Seoul 04763 (KR)**

(74) Representative: **Stolmár & Partner
Patentanwälte PartG mbB
Blumenstraße 17
80331 München (DE)**

Remarks:
The complete document including Reference
Table(s) and the Sequence Listing(s) can be
downloaded from the EPO website

(54) **PHARMACEUTICAL COMPOSITION FOR ALLEVIATING OR TREATING SEQUELAE OF CEREBRAL INFARCTION**

(57) The present invention provides a pharmaceutical composition for alleviation or treatment of cerebral infarction sequela, which includes glia-like cells differentiated from human mesenchymal stem cells (hMSCs). The pharmaceutical composition of the present invention is preferably used as a therapeutic agent for alleviating or treating one or more cerebral infarction sequela selected from the group including paralysis, dysphagia, cognitive impairment, aphasia and stuttering.

[FIG. 1b]

**EP 4 356 916 A1**

**Description**

[Technical Field]

The present invention relates to a pharmaceutical composition for alleviation or treatment of cerebral infarction sequela (or aftereffect), which includes glia-like cells differentiated from human mesenchymal stem cells (hMSCs).

[Background Art]

The nervous system consists of nerve cells (neuron) and glia cells. Glia cells are cells that support the nervous system, make up about 90% of the nervous system, supply necessary substances to neurons, and maintain homeostasis for a suitable chemical environment. Unlike neurons that transmit information, the glia cells do not have an information transfer function. Further, unlike neurons, these can recover after loss. Therefore, cancer occurring in the brain is caused by the glia cells rather than neurons. Glia cells are the most distributed cells in the brain, and a size of the glia cells is about 1/10 the size of the nerve cells, but the number thereof is estimated to be about 10 times the number of neurons, that is, hundreds of billions. The glia cells present in the central nervous system may include: astrocytes which maintain the blood-brain barrier, absorb glucose from the blood thus to supply the same to neurons, help tissue regeneration and improve the microenvironment; oligodendrocytes which form central nervous system myelin sheath; and microglia and radial glia which act as immune cells of the central nervous system. Further, glia cells present in the peripheral nervous system may include: Schwann cells which form peripheral nervous system myelin sheaths; and satellite cells which supply nutrients to the neurons.

Bone marrow-derived mesenchymal stem cells (MSCs) are capable of self-renewal while maintaining growth in a laboratory, and can differentiate into various cells. Further, MSCs have the potential to cross-differentiate into a variety of neuron-like cells with neuronal activity. Human mesenchymal stem cells (hMSCs) are being studied as a cell therapy for treating the damaged nervous system due to high plasticity and low immune rejection. The present inventors demonstrated that transplanted hMSCs increase the growth of damaged nerve fibers and survival of spinal cord tissue cells in an ex *vivo* spinal cord injury model.

Stem cell therapy may induce recovery of the damaged nerve system in the intractable nervous disease, thus being assessed as a promising therapeutic method. Mesenchymal stem cells of adult stem cells, which are mainly used in the stem cell therapy, include early passage hMSC (hMSC) in the early passage of culture and late passage hMSC (10 passages or more) in the late passage of culture. The hMSC in the late passage of culture secretes less growth factors or cytokines, which in turn has decreased paracrine effects to reduce the recovery of the nervous system. Accordingly, initial hMSCs are used for treatment of the damaged nervous system. However, since an amount of hMSCs obtainable in the initial stage of culture is very limited, it is difficult to obtain a sufficient amount of hMSCs for treatment. Further, great expense is required and cannot increase the possibility of use.

Cerebral infarction refers to a neurological symptom occurring when a blood vessel supplying blood to a portion of the brain is blocked. The stroke caused due to the cerebral infarction corresponds to a disease accounting about 85% of the total stroke. For treatment of cerebral infarction, it is important to recover the damaged nervous system. However, since the nerve cells, that is, neurons are not recovered if damaged once, it is important to recover the function of glia cells. Among the glia cells, it is reported that astrocytes secreting growth factors to improve the microenvironment are the most helpful in the treatment of cerebral infarction.

The cerebral infarction sequela, which means, after a cerebral infarction-based pathogenic mechanism is completed, a chronic cerebral disease accompanied with neurological aftereffect due to previously dead nerve cells without additional damage in cerebral cells, corresponds to an intractable disease to which no therapeutic method is currently applied. In this circumstance, although development of different treatment methods for such chronic aftereffects of the cerebral infarction have been attempted till now, all failed. The reason for this result is expected because lots of cerebral tissues are simultaneously damaged by different mechanisms in the case of cerebral infarction, and only single target therapeutics are used for improving aftereffects thereof. In regard to this limitation, a stem cell therapeutic having various mechanisms concurrently now emerges as an alternative.

[Summary of Invention]

[Problems to be solved by Invention]

An object of the present invention is to provide a pharmaceutical composition for alleviation or treatment of cerebral infarction sequela, which includes glia-like cells.

[Means for Solving Problems]

1. A pharmaceutical composition for alleviation or treatment of cerebral infarction sequela, comprising glia-like cells differentiated from human mesenchymal stem cells (hMSCs).

2. The pharmaceutical composition according to the above 1, wherein the glia-like cell expresses one or more marker genes selected from the group consisting of GFAP, GLAST, HGF, VEGF, IGFBP-4 and PAPP-A.

3. The pharmaceutical composition according to the above 1, wherein the human mesenchymal stem cell is derived from tissues selected from the group including bone marrow, fat, peripheral blood, cord blood, muscle and skin.

4. The pharmaceutical composition according to the above 1, wherein the human mesenchymal stem cell is passage-cultured twice to 15 times.

5. The pharmaceutical composition according to the above 1, wherein the cerebral infarction sequela is one or more selected from the group including paralysis, dysphagia, cognitive impairment, aphasia and stuttering.

[Advantageous Effects]

The pharmaceutical composition for alleviation or treatment of cerebral infarction sequela, which includes glia-like cells differentiated from mesenchymal stem cells (MSCs), may induce regeneration of cerebral cells or glia cells, or inhibit damage to the same. Preferably, the pharmaceutical composition may be used for suppressing aftereffects caused by cerebral infarction such as paralysis, dysphagia, cognitive impairment, aphasia, stuttering, or the like, or for alleviating or treating the progress of the aftereffects.

[Brief Description of Drawings]

FIGS. 1a to 1d illustrate the confirmation of morphological variation of glia-like cells during induction thereof, and an expression level of a marker gene of the glia cell.

FIG. 2 is a schematic view illustrating an experimental plan in order to identify whether to alleviate the cerebral infarction sequela or not by the glia-like cells.

FIG. 3 is a schematic view indicating a blocked site in the middle cerebral artery blockage operation in order to induce cerebral infarction.

FIG. 4 illustrates measured results of body weight change in rats over time after inducing the cerebral infarction.

FIGS. 5a and 5b illustrate MRI observed results of the brain of rats with or without glia-like cell treatment after induction of cerebral infarction.

FIGS. 6a and 6b illustrate measured results of the change in volume of the cerebral infarction with or without glia-like cell treatment after induction of cerebral infarction.

FIGS. 7a to 7c illustrate results of sticky tape test with or without glia-like cell treatment after induction of cerebral infarction.

FIGS. 8a and 8b illustrate results of inclined plane test with or without glia-like cell treatment after induction of cerebral infarction.

FIGS. 9a and 9b illustrate measured results of change in volume of the cerebral infarction with or without glia-like cell treatment after induction of cerebral infarction.

FIGS. 10a and 10b illustrate measured results of inclined plane test with or without glia-like cell treatment after induction of cerebral infarction.

FIG. 11 illustrates results of quantitatively confirming differentiation marker of ghMSC transplanted in a cerebral infarction sequela rat model through real-time qPCR method in mRNA level.

FIG. 12 illustrates results of quantitatively measuring concentrations of VEGF and IGBP-4 proteins of hMCS-CM and ghMSC-CM through ELISA.

FIG. 13 illustrates flow cytometry assay results in ghMSC.

FIG. 14 illustrates immunocytochemistry (ICC) results of hMSC and ghMSC.

FIGS. 15a to 15c illustrate cytokinecytokine array results of hMSC and ghMSC.

FIGS. 16a to 16f illustrate schematic views of ex *vivo* cerebral infarction sequela experiments and confirmation of cell death protection effects, wherein (a) is an experimental schematic view; (b) illustrates cortex slice; (c) is a cell death image of cortex sites stained with (A) PI; (d) illustrates cell death rate; (e) is a cell death image of cortex sites stained with PI; and (f) illustrates cell death rate.

FIGS. 17a and 17b illustrate results of confirming transplantation effects in ghMSC tissues in ex *vivo* Chronic Stroke Brain Slice. After confirming and treating with CXCR2 antagonist (SB225002), cell death image of transplanted cells in ghMSC tissue; (a) MAP2 image in cortex site as a neuronal marke through IHC staining; and (b) MAP2 fluorescence intensity are illustrated.

FIG. 18 illustrates a schematic view of *in vitro* cerebral infarction sequela experiments, and pAkt expression results in ReN VM cells with OGD induced using glia-like cells and an adult stem cell culture solution, wherein (a) is an experimental schematic view, (b) illustrates pAkt expression confirmed through Western blotting, and (c) is a pAkt quantitative graph.

FIG. 19 illustrates confirmation of transplantation effects in ghMSC tissue through TUNL assay in ex *vivo* Chronic Stroke Brain Slice and confirmation of transplanted cell death in ghMSC tissue after treatment with CXCR2 antagonist (SB225002), wherein (a) is cell death image in cortex site through TUNEL staining, and (b) is a cell death graph.

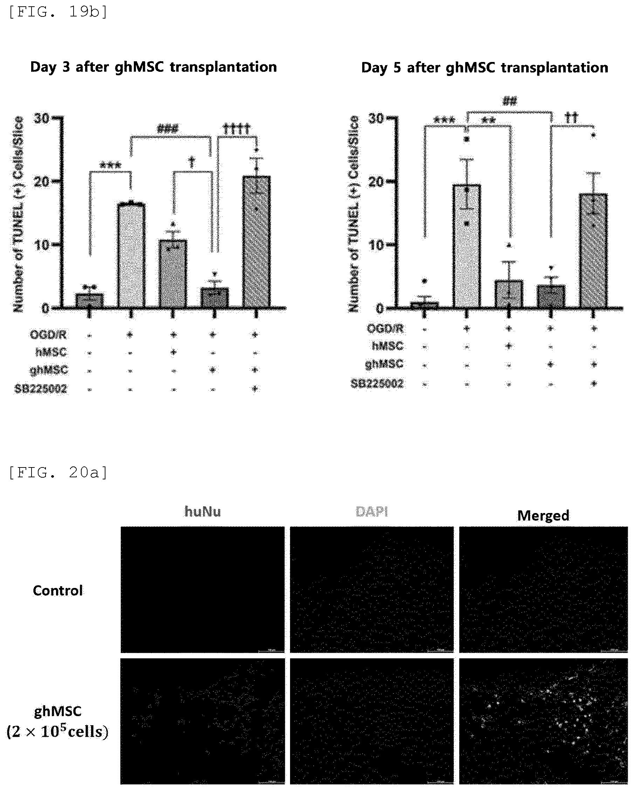

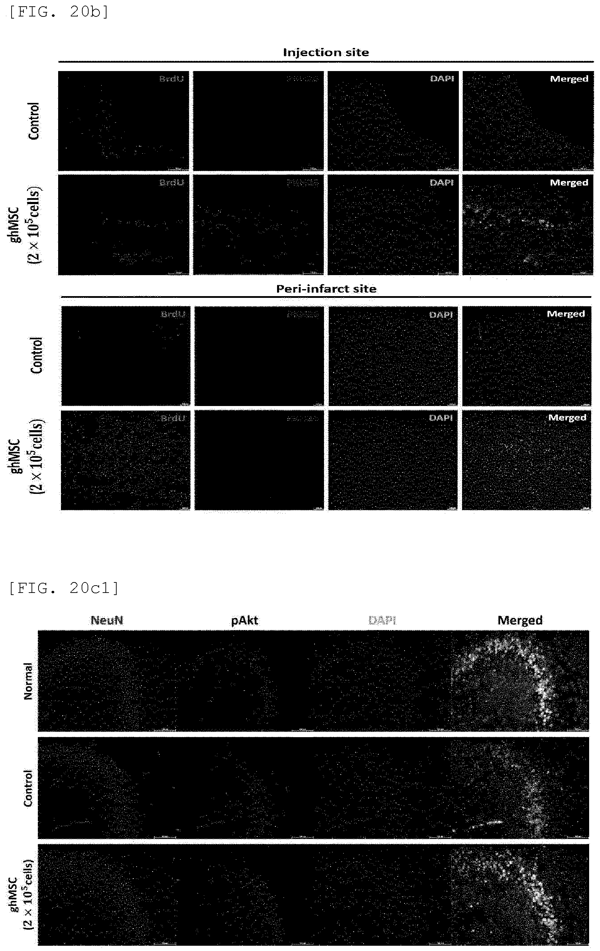

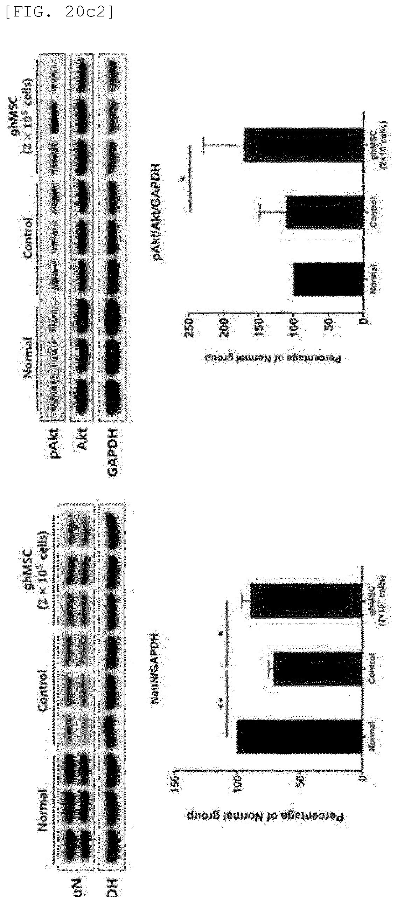

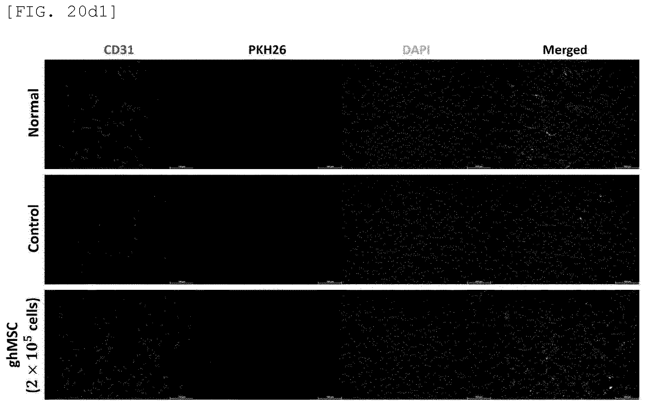

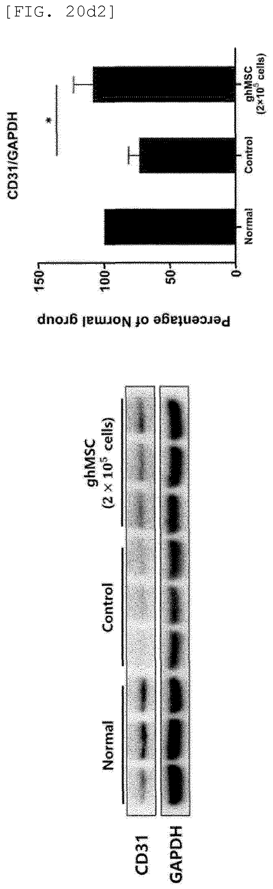

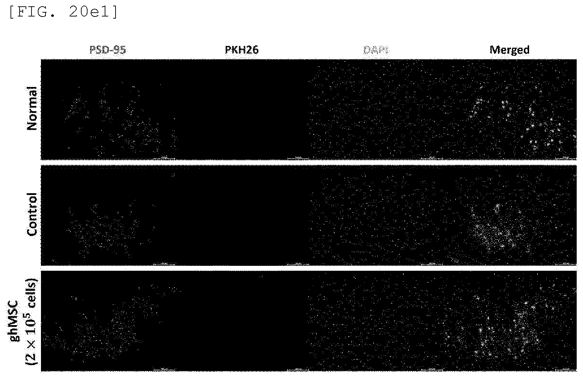

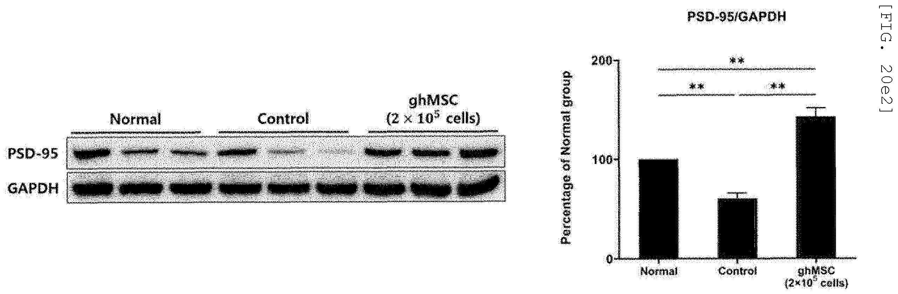

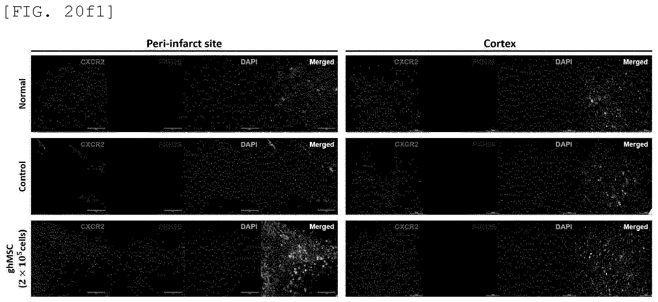

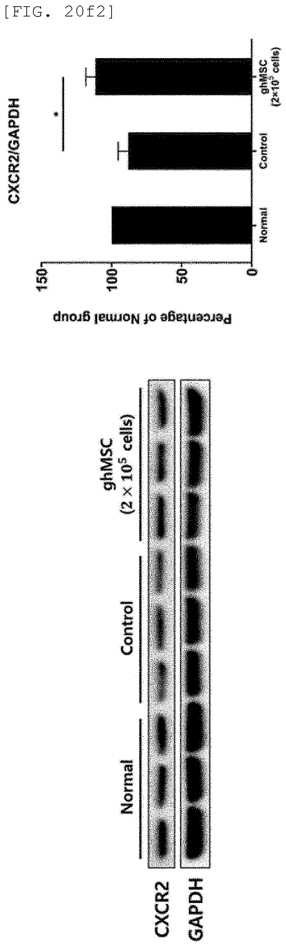

FIG. 20 illustrates results of molecular biological assessment using brain tissues in a cerebral infarction sequela model transplanted with ghMSC and neurobsal media, wherein (a) is ghMSC residual image in an administered site using human nuclei antibody; (b) illustrates BrdU expression labeling proliferated cells confirmed through IHC; (c) illustrates pAkt and NeuN expression through IHC and Western blotting; (d) is IHC image and Western blot graph of CD31 exhibiting vascular endothelial cells; (e) is IHC image and Western graph of PSD-95 protein relevant to synaptic plasticity; and (f) is CXCR2 expression image and Western blot graph relevant to ghMSC mechanism.

[Mode for Carrying out Invention]

Hereinafter, the present invention will be described in detail. Unless otherwise specifically defined, all terms in the present specification would have the same meanings as general meanings of the corresponding terms understood by persons having common knowledge to which the present invention pertains ("those skilled in the art"), and if the general meanings conflict with the meanings of the terms used herein, the meanings used in the present specification take precedence.

The present invention relates to a pharmaceutical composition for alleviation or treatment of cerebral infarction sequela, which includes glia-like cells differentiated from human mesenchymal stem cells (hMSCs).

In the present invention, the glia-like cell corresponds to a cell expressing a marker gene of glia cell, in particular, astrocyte, or a cell secreting growth factor or cytokine secreted from the astrocyte. The marker gene may correspond to GFAP, GLAST, HGF, VEGF, IGFBP-4 or PAPP-A, while the glia-like cell may refer to cells relatively highly expressing at least one or more among the marker genes, as compared to undifferentiated stem cells. The lower limit of mRNA expression level of the marker gene in the glia-like cells may be 1.1, 1.2, 1.3, 1.5 or 2 times as compared to the undifferentiated stem cells, while the upper limit of mRNA expression level of the marker gene in the glia-like cells may be 20, 15, 10, 8, 6 or 3 times as compared to the undifferentiated stem cells, but they are not limited thereto.

The glia-like cells according to the present invention are differentiated from hMSCs, and any glia-like cell is included in the scope of the present invention without limitation thereof in terms of the preparation method, so far as it can express the marker gene. Examples of the preparation method may include a process consisting of: primary culture of hMSCs in a medium containing $\beta$-mercaptoethanol; secondary culture of the primary culture product in a medium containing all-trans-retinoic acid; and tertiary culture of the secondary culture product in a medium containing human basic fibroblast growth factor (hbFGF), human platelet derived growth factor AA (hPDGF-AA), forskolin and heregulin-$\beta 1$ (HRG-$\beta 1$) to produce the final product, but they are not limited thereto.

In the primary culture, the mercaptoethanol may be included in an amount of 0.05 to 20 mM, 0.1 to 10 mM, 0.2 to 5 mM or 0.5 to 2 mM based on the total primary culture solution. Further, in the secondary culture, the all-trans-retinoic acid may be included in an amount of 0.01 to 2 $\mu$g/mL, 0.05 to 1 ug/mL or 0.1 to 0.5 $\mu$g/mL based on the total secondary culture solution. Further, in the tertiary culture, the human basic fibroblast growth factor (hbFGF) may be included in an amount of 0.01 to 1 ng/mL, 0.1 to 100 ng/mL, 1 to 50 ng/mL or 5 to 20 ng/mL based on the total tertiary culture solution. Likewise, the human platelet derived growth factor AA (hPDGF-AA) may be included in an amount of 0.1 to 100 ng/mL, 0.5 to 20 ng/mL or 1 to 10 ng/mL, the forskolin may be included in an amount of 0.5 to 100 $\mu$M, 1 to 50 $\mu$M or 5 to 20 $\mu$M, and the heregulin-$\beta 1$ (HRG-$\beta 1$) may be included in an amount of 1 ng/mL to 1 $\mu$g/mL, 10 to 500 ng/mL or 50 to 300 ng/mL, but they are not limited thereto. In addition, the culture solution may further include a composition such as DMEM or FBS to provide nutrients commonly used in cell culture, antibacterial/antiviral substances such as a mixture of penicillin/streptomycin, or the like, but it is not limited thereto.

As the cerebral infarction sequela in the present invention may include, any aftereffect occurring in the body is included in the scope of the present invention without limitation thereof so far as the aftereffect is caused by damage to cerebral cells or glia cells or death thereof due to stroke, cerebral infarction, cerebral hemorrhage, etc. For example, paralysis, dysphagia, cognitive impairment, aphasia, stuttering, or the like may be included.

The pharmaceutical composition of the present invention may promote the regeneration of neural stem cells that may be differentiated into corresponding cerebral cells at a site in which the cerebral cells including nerve cells, astrocytes or oligodendrocytes are damaged and/or dead, or induce improvement of brain functions, whereby the cerebral infarction sequela can be alleviated or treated. Specifically, a volume of cerebral infarction may be reduced by the glia-like cell itself or growth factor/cytokine secreted from the glia-like cell, and thereby the progress of aftereffects such as paralysis, dysphagia, cognitive impairment, aphasia, stuttering, or the like as described above may be inhibited, or further, even symptoms thereof may be improved. The composition of the present invention may promote the generation of the damaged neural stem cells or induce the improvement of damaged functions as described above, therefore, may have a function distinguishable from the prevention of damage occurring due to causes of the cerebral infarction such as ischemia. Further, even after removing the above causes, the residual damage may be improved.

As the human mesenchymal stem cell (hMSC) in the present invention, any hMSC may be included in the scope of the present invention regardless of tissues from which the hMSC is derived so far as it can be induced into glia-like cell by the culture process as described above. For example, it may be derived from tissues selected from the group including bone marrow, fat, peripheral blood, cord blood, muscle and skin, but it is not limited thereto.

The hMSC may be differentiated into glia-like cells and used in the present invention regardless of the number of passage cultures. For example, primary culture line may be used, or hMSCs passage-cultured once or more may be used. Further, even the late-passage stem cells, which were passage-cultured 9 times or more, may be induced into glia-like cells showing effects of alleviating the cerebral infarction sequela described above. Specifically, the lower limit of the number of passage cultures is not limited but may be 1, 2, 3 or 4 times. On the other hand, the upper limit of the number of passage cultures may be 20, 15, 12, 11, 10, 9 or 8 times, but it is not limited thereto.

The pharmaceutical composition of the present invention may include active ingredients alone, or further include at least one pharmaceutically acceptable carrier, excipient or diluent to be provided as a pharmaceutical composition. Further, a substance having similar efficacy known in the art may also be administered in combination with the cells, but it is not limited thereto.

An administration route of the pharmaceutical composition according to the present invention may include oral, intravenous, intramuscular, intra-arterial, intra-medullary, intradural, intracardiac, transdermal, subcutaneous, intraperitoneal, intranasal, intestinal, local, sublingual or intrarectal route, but it is not limited thereto.

The pharmaceutical composition of the present invention may be administered orally or parenterally and, in the case of parenteral administration, skin application or intraperitoneal, intrarectal, subcutaneous, intravenous, intramuscular or intrathoracic injection method may be selected for administration, but it is not limited thereto.

A preferable administration amount of the pharmaceutical composition may vary depending on the condition and body weight of the affected subject, the severity of disease, the form of the drug, the route and duration of administration, but may be appropriately selected by those skilled in the art. For example, it may be administered so that the number of administered cells per 1 kg of body weight reaches $1 \times 10^3$ to $1 \times 10^9$ cells/kg, preferably $1 \times 10^4$ to $1 \times 10^8$ cells/kg, and more preferably $1 \times 10^5$ to $1 \times 10^7$ cells per 1 kg body weight, but it is not limited thereto. Instead, it may vary and be administered depending on the condition of the patient and the degree of onset of disease. Further, the administration may be once or divided into several times a day. Further, the administration dosage does not limit the scope of the present invention in any way.

The pharmaceutical composition of the present invention may be formulated in a unit dose form using any pharmaceutically acceptable carrier and/or excipient, or may be formulated by introducing the same into a multi-dose container according to any method easily implemented by those skilled in the art, to which the present invention pertains. At this time, the formulation may have the form of a solution, suspension or emulsion in oil or aqueous medium, or the form of extract, powder, granules, tablets or capsules, and may further contain a dispersing agent or stabilizer.

Hereinafter, the present invention will be described in detail and illustrated by means of the following examples.

**Example 1**

**1. Culture of human mesenchymal stem cells (hMSCs)**

Adult human mesenchymal stem cells (hMSCs) extracted from normal human bone marrow (Cambrex Bioscience, Walkersville, MD, USA) were cultured in a low-glucose Dulbecco's modified Eagle's medium (DMEM, low glucose) containing 10 % FBS (Gibco, Waltham, MA, USA) added thereto. Cells (passages 6-12) were subjected to passage culture at 37 °C under 5 % $CO_2$ environment for 12 to 15 times thus to obtain late-passage hMSCs.

**2. Induction of human mesenchymal stem cell into glia-like cell (glial-like cell induced from human mesenchymal stem cell, ghMSC)**

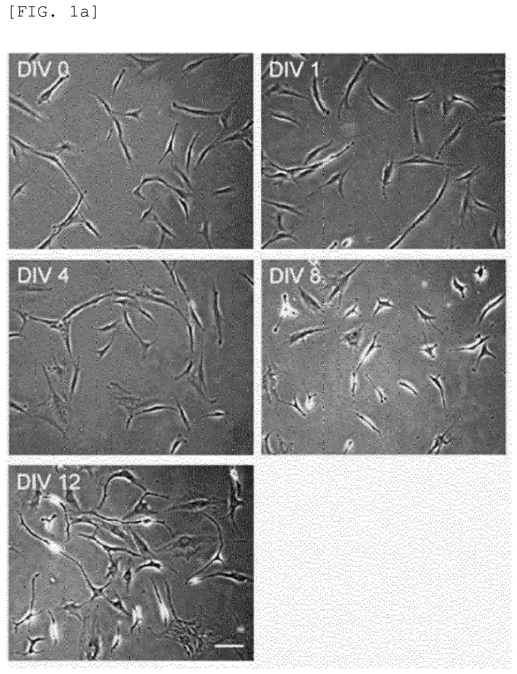

The late-passage hMSCs obtained in Example 1 above were cultured for 24 hours while exchanging the medium with primary-differentiated medium [DMEM-low glucose medium, 10 % FBS, 1 % penicillin-streptomycin mixture, 1 mM β-mercaptoethanol]. Thereafter, the hMSCs were washed with PBS (phosphoric acid buffer solution), and then cultured while exchanging the medium with secondary differentiated medium [DMEM-low glucose medium, 10 % FBS, 1 % penicillin-streptomycin mixture, 0.28 μg/mL all-trans-retinoic acid]. After 3 days, the MSCs were washed with PBS and then cultured for 8 days while exchanging the medium with tertiary differentiated medium [DMEM-low glucose medium, 10 % FBS, 1 % penicillin-streptomycin mixture, 10 μM forskolin, 10 ng/mL human basic-fibroblast growth factor, 5 ng/mL human platelet derived growth factor-AA, 200 ng/mL heregulin-β1]. At this time, the tertiary differentiated medium was exchanged once every other day. For differentiation induction, the form of hMSC under culture was observed by a microscope. The survival of cells during differentiation induction was observed through an optical microscope (FIG. 1a).

### 3. Preparation of concentrated culture solution of glia-like cells

Glia-like cells ($1 \times 10^4$ cells/cm$^2$) were washed with PBS twice, and then cultured for 18 hours with serum-free Neurobasal-A medium (NB medium). After then, the concentrated culture solution was collected and precipitated by means of a centrifuge at 1,500 g for 5 minutes to remove cell waste, followed by using the same in experiments.

### 4. Freezing and thawing of glia-like cells

#### (1) Freezing of glia-like cells

1-2 $\times$ 10$^5$ hMSCs (or glia-like cells) in DMEM medium containing 10% FBS, FBS crude and DMSO were prepared in a ratio of 5:4:1 to reach 1 mL volume, thereby preparing a freezing vial. Then, the prepared freezing vial was placed in a freezing container containing 100% isopropanol, left in a deep freezer at -80 °C overnight, and then, placed in a liquid nitrogen storage tank (LN2 tank) for storage.

#### (2) Thawing of glia-like cell

The freezing vial stored in the liquid nitrogen storage tank was thawed in a water bath at 37 °C for 2 minutes, cells slightly including ice in the freezing vial were well mixed with a pipette in a biosafety hood. 1 mL of cell concentrate in the freezing vial was admixed with 10 mL of DMEM medium containing 10% FBS and placed in 15 mL conical tube. After centrifuging at a speed of 1,200 rpm for 5 minutes, all supernatants were suctioned while remaining the precipitated cell pellets. Then, 1 mL of DMEM medium containing 10% FBS was further added to the 15 mL conical tube and the cell pellets were dispersed with the pipette. 11 μL of cell pellets was admixed with a dye, that is, 11 μL of trypan blue. 10 μL of the mixture was introduced to a cell counter to calculate the number of cells contained in 1 mL of the mixture, followed by spreading the same at 2,000 cells/cm$^2$ on a cell culture dish.

### 5. Analysis of characteristics of glia-like cells induced from late-passage hMSCs

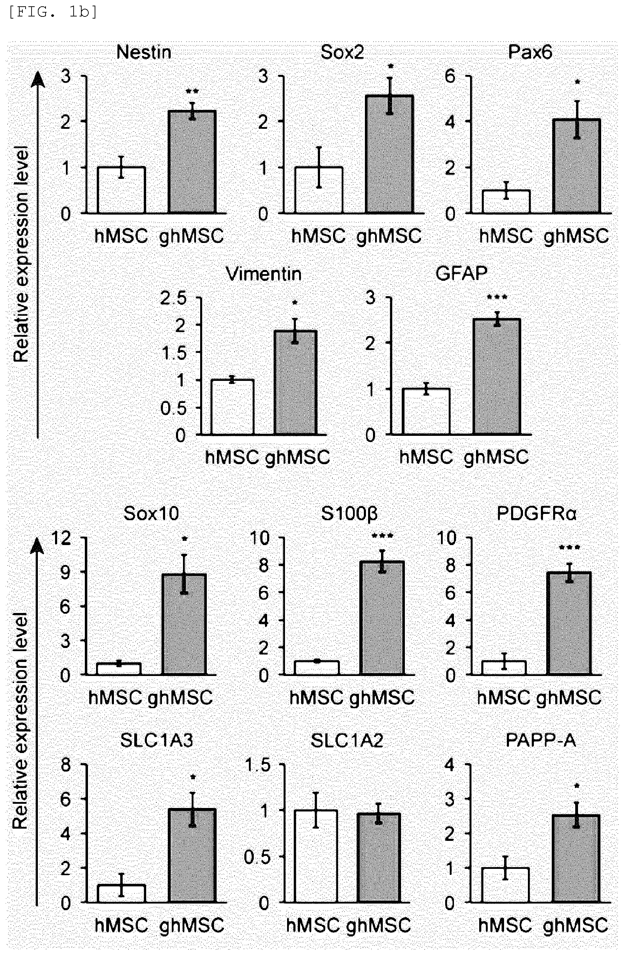

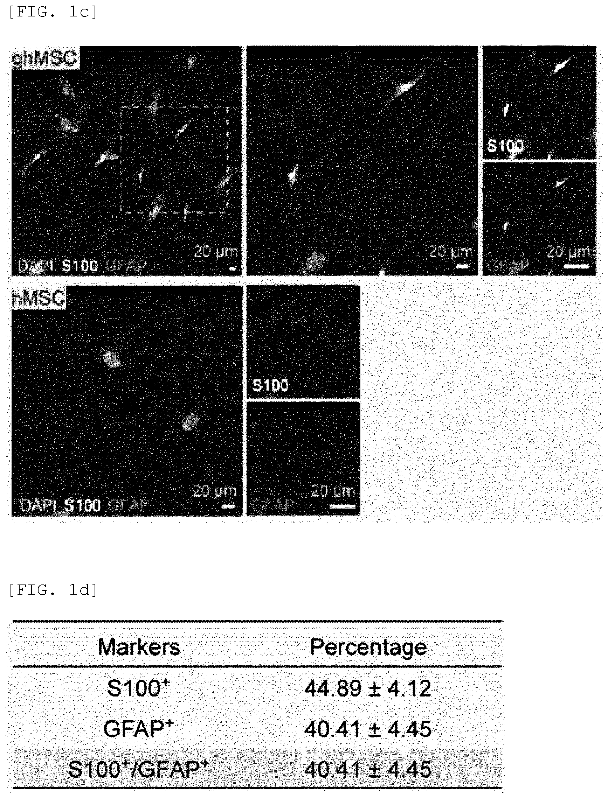

To confirm the characteristics of glia-like cells derived from hMSCs induced by the method in Example 2, a degree of expression of glia cell marker was determined through quantitative RT-PCR and immunohistochemical staining.
Specifically, according to the quantitative RT-PCR, RNA was extracted using TRIzol reagent (Invitrogen, Carlsbad, CA, USA), and then treated with DNase. For cDNA synthesis, reverse transcription was performed using M-MLV reverse transcriptase (Promega) at 42 °C for 1 hour. Using SYBR FAST qPCR Kits (KAPA Biosystems), genes expressed in cDNA were confirmed with gene-specific primers having SEQ ID NOs: 1 to 24, respectively. The degree of expression of a target gene was determined with reference to GAPDH using Ct method. △Ct value is a vale obtained by subtracting Ct value, while a cycle number of critical value was measured as (△Ct=Ct (Target)-Ct(GAPDH)). A relative value of target gene to endogenous GAPDH was determined by fold-change of GAPDH=$2^{-\triangle Ct}$.
The immunohistochemical staining was performed to fix the cultured cells with 4% paraformaldehyde (PFA), followed by culturing the cells with 5% standard chlorine serum and 0.1% Triton-X100 blocking solution. The product was stained with GFAP (1:200; Merck millipore), S100 (1:250; Dako) in a refrigerator at 4 °C for 1 day. Then, the stained solution was washed several times with PBS, followed by staining the cells with the secondary antibody, Alexa Fluor$^®$488 anti-mouse IgG (Molecular Probes) and Alexa Fluor$^®$546 anti-rabbit IgG (Molecular Probes) at room temperature for 1 hour. Thereafter, a nucleus was stained with 4,6-diamidino-2-phenylindole (DAPI; Santa Cruz Biotechnology). The sample was observed using a digital inverted fluorescence microscope (DM5000B; Leica).
As a result, it was confirmed that the expression of a radial glia marker such as Nestin, Sox2, Pax6, Vimentin or GFAP, an astrocyte marker such as FAP, S100β and SLC1A3, an oligodendrocyte marker such as Sox10, etc. is more increased in ghMSC than hMSC. Further, it was confirmed that ghMSC of IGF-1 pregnancy-associated plasma protein-A (PAPP-A) as well as IGFBP-4 shows more increased expression in ghMSC than hMSC (FIG. 1b). Further, as shown in FIGS. 1c and 1d, it was confirmed that Sox10 and GFAP as the markers of oligodendrocyte were expressed by 45% and 40%, respectively, in the total ghMSCs.

**6. Confirmation of therapeutic effects by ghMSC in cerebral infarction sequela model**

**(1) Construction of cerebral infarction sequela animal model**

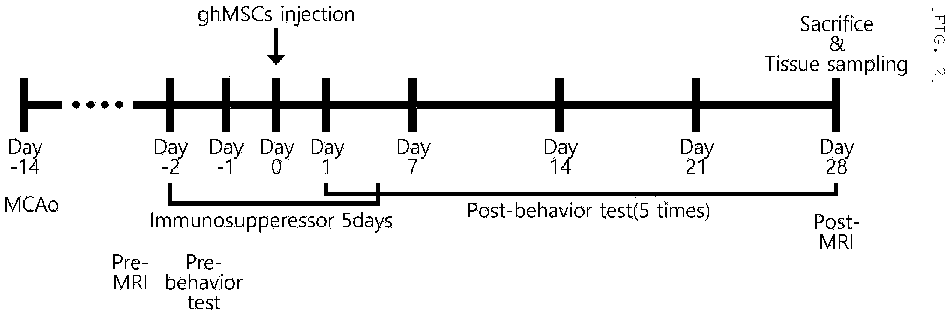

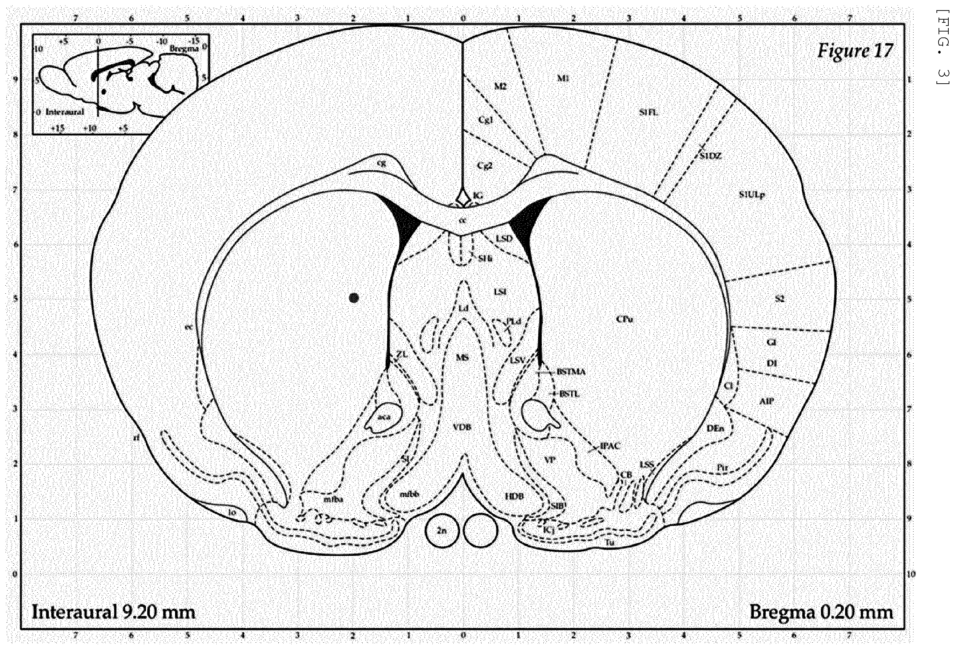

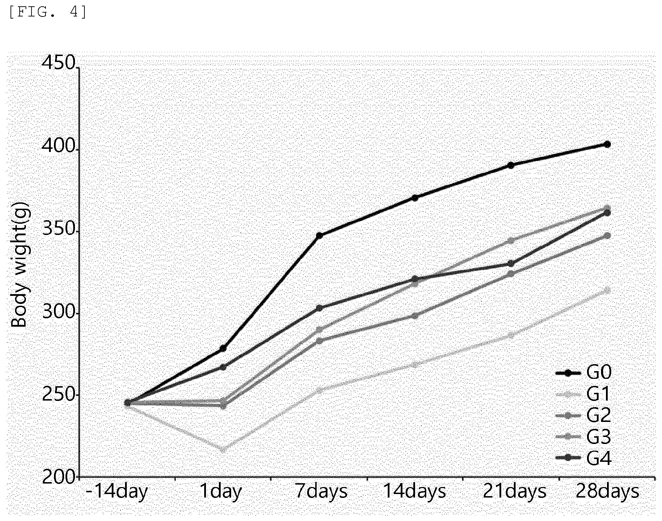

Hereinafter, all animal experiments were executed in compliance with ethical treatment of experimental animal and rules for scientific use (LARL-2022-0017, LARL-2022-0003). Approximate experiment plans for cerebral infarction sequela model are illustrated in FIG. 2. 8-week old male SD rats with 250 g $\pm$ 25 g body weight were subjected to middle cerebral artery appendage occlusion. Specifically, a 4-0 silicon-coated nylon thread is inserted through the internal carotid branched from the common carotid to block the middle cerebral artery for 2 hours, followed by removing the thread to allow reperfusion, thereby inducing acute cerebral infarction (FIG. 3). Thereafter, this experimental group was used by 2-week interval, in order to prepare a cerebral infarction sequela animal model. Due to a high mortality rate after the middle cerebral artery appendage occlusion, the condition of animal was visibly confirmed once a day. From 2 weeks after the preparation of cerebral infarction sequela animal model till autopsy, the animal was subjected to body weight measurement once per week. As a result, a change in body weight by a certain period of time was around $\pm 15$ % in all groups, therefore, no significant change in body weight was observed (FIG. 4).

**(2) Configuration of experimental group and administration of ghMSC**

To confirm an efficacy of ghMSC in the cerebral infarction sequela model and establish an optimum amount thereof, experimental groups were prepared in the range of different amounts. With reference to the maximum amount used in the existing studies, $2 \times 10^5$ cells, the number of cells (amount) was reduced by a geometric ratio of 0.3, and thus set to G2-G4, followed by carrying the corresponding cells in neurobasal media (Neurobasal-A medium; Gibco). Further, an excipient control group, G1, to which the excipient only was administered, was prepared (Table 1).

[TABLE 1]

| Group | Total cell number | Amount |
|---|---|---|
| G0 | 0 | 0 $\mu$L |
| G1 | 0 | 15 $\mu$L |
| G2 | $2 \times 10^5$ | 15 $\mu$L |
| G3 | $6 \times 10^4$ | 15 $\mu$L |
| G4 | $2 \times 10^4$ | 15 $\mu$L |

The red point in FIG. 3 indicates striatum at peri-infarct site around ischemic core, which was injected into the corresponding site as a target. To each of G0 to G4 groups, 15 $\mu$L of solution was first administered at a flow rate of 1 $\mu$L/min.

**(3) MR Image photographing and assay**

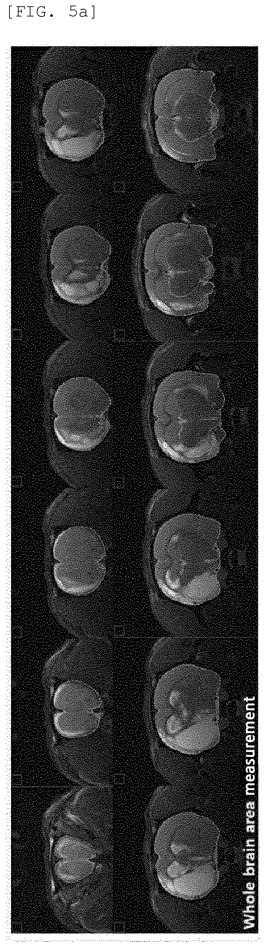

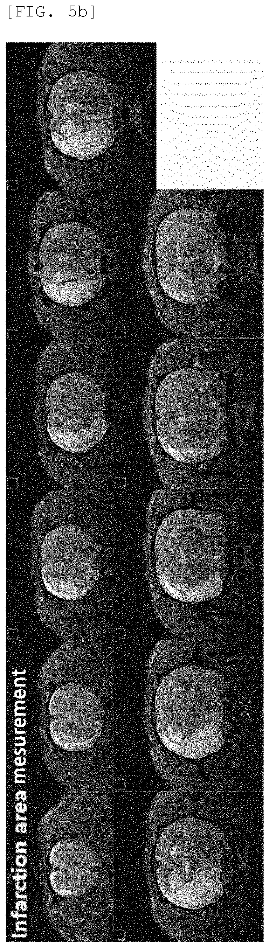

An injection date of ghMSC into the cerebral infarction sequela animal model was set as day 0, and then MR image was photographed on day 2 and day 28, respectively, followed by evaluating improvement of a cerebral infarction volume using values analyzed through image J software. Specifically, the whole brain area was measured, followed by measuring an area with cerebral infarction confirmed in MR data thus to calculate the cerebral infarction volume (%) in percentage of cerebral infarction area/whole brain area. After photographing 2 days before cell administration, subjects with 15 to 25% cerebral infarction volume were uniformly divided into individual groups. Then, 28 days after cell administration, the groups were photographed to analysis a change in the cerebral infarction volume (FIGS. 5a and 5b).

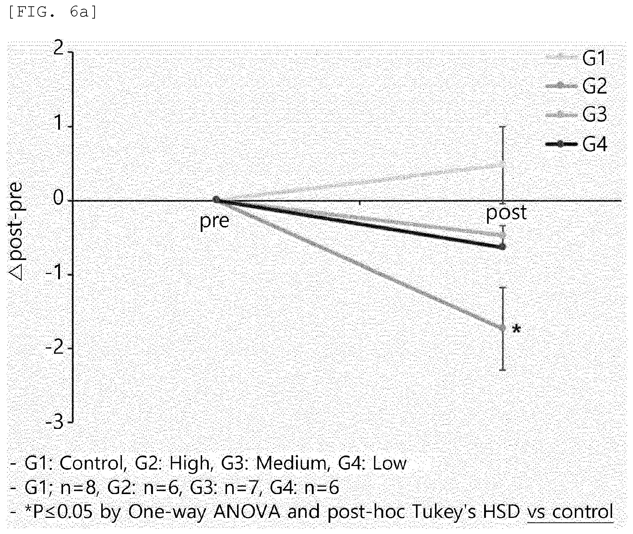

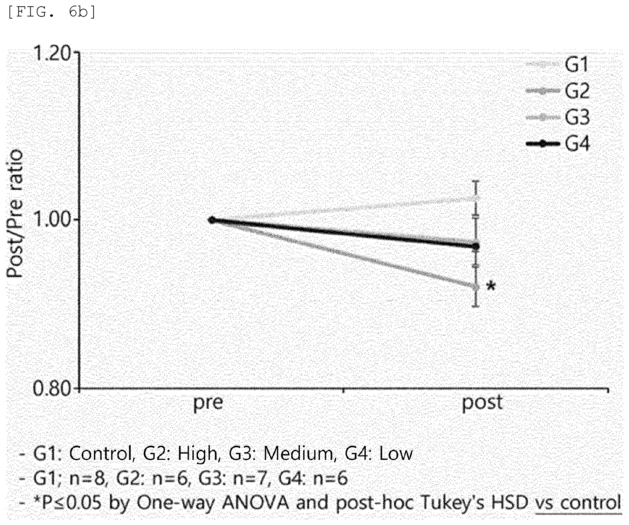

Thus, as a result of comparing the changes after cell administration in the groups, it was confirmed that G1: $0.48 \pm 0.40$, G2: $-1.74 \pm 0.52$, G3: $-0.48 \pm 0.56$, G4: $-0.64 \pm 0.14$, and therefore, G2 group shows statistically significant results compared to G1 group (*P<0.05) (FIG. 6a). Further, as a result of comparing the ratio of cerebral infarction volume after cell administration divided by cerebral infarction volume before cell administration, it was confirmed that G1: $1.03 \pm 0.02$, G2: $0.92 \pm 0.02$, G3: $0.97 \pm 0.03$, G4: $0.97 \pm 0.01$, and therefore, G2 group showed statistically significant results compared to G1 group (*P<0.05) ( 도 6b) .

**(4) Sticky tape test**

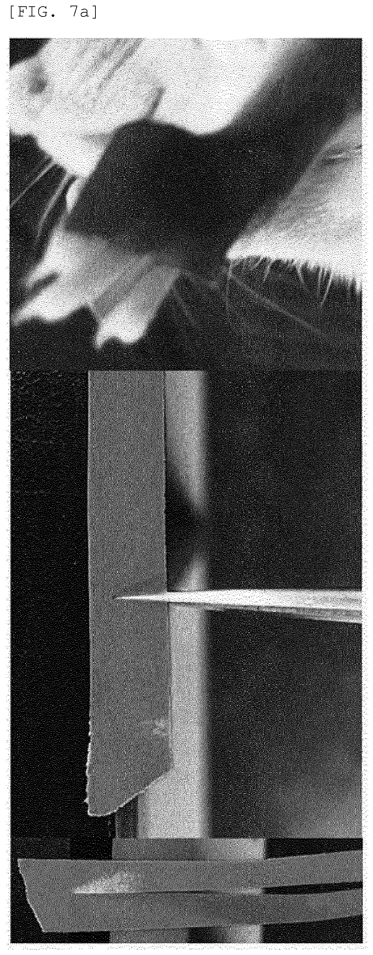

After ghMSC administration, sticky tape test was executed on day 1, 7, 14, 21 and 28. The date of administering ghMSC to the cerebral infarction sequela model was set as day 0, and then the sticky tape test was continued till day 1, 7, 14, 21 and 28 after administration. Specifically, 3M labeled tape was vertically cut by half, followed by cutting the tape in a size of 0.75 cm length and 2 cm width. Then, the tape was attached to each of paws of a rat so that the tape was sticky on the palm. Thereafter, a time taken to detach the tape after recognizing the same was measured while recording a time of recognizing the tape (FIG. 7a).

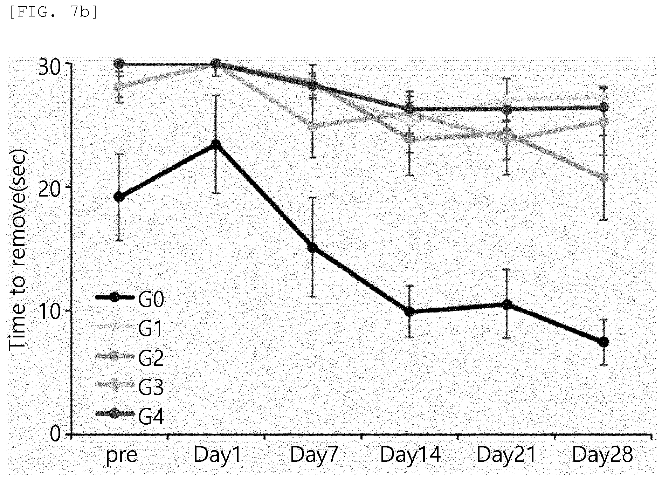

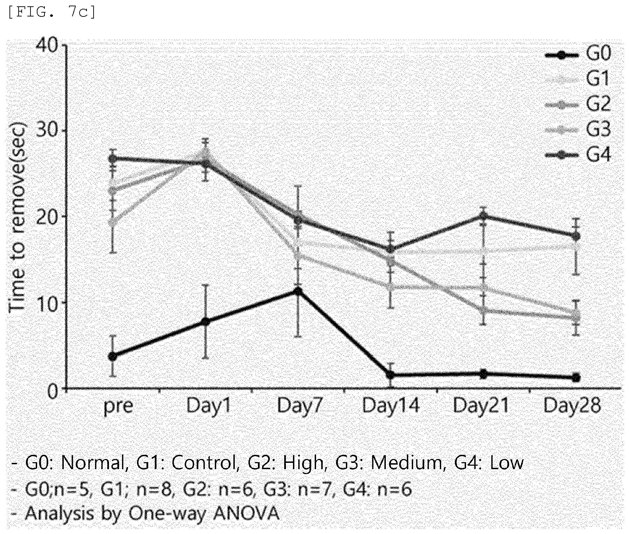

As a result, the time of detaching the tape in individual groups 28 days after cell administration was confirmed as follows: G0 : 7.47 ± 1.84, G1 : 27.29 ± 0.83, G2 : 20.78 ± 3.40, G3 : 25.29 ± 2.68, G4 : 26.45 ± 1.67. Although statistically significant difference was not demonstrated, it was confirmed that G2 group and G3 group have the tendency of decreasing the time compared to G1 group. The measured time for recognition in individual groups was confirmed as follows: G0 : 1.27 ± 0.47, G1 : 16.5 ± 3.30, G2 : 8.17 ± 2.02, G3 : 8.76 ± 1.36, G4 : 17.78 ± 4.04. Although statistically significant difference was not demonstrated, it was confirmed that G2 group and G3 group have the tendency of decreasing the time for recognition of the tape compared to G1 group. G0 group is a normal group and showed statistically significant difference compared to G1 group (P<0.05) (FIGS. 7b and 7c).

**(5) Inclined plane test**

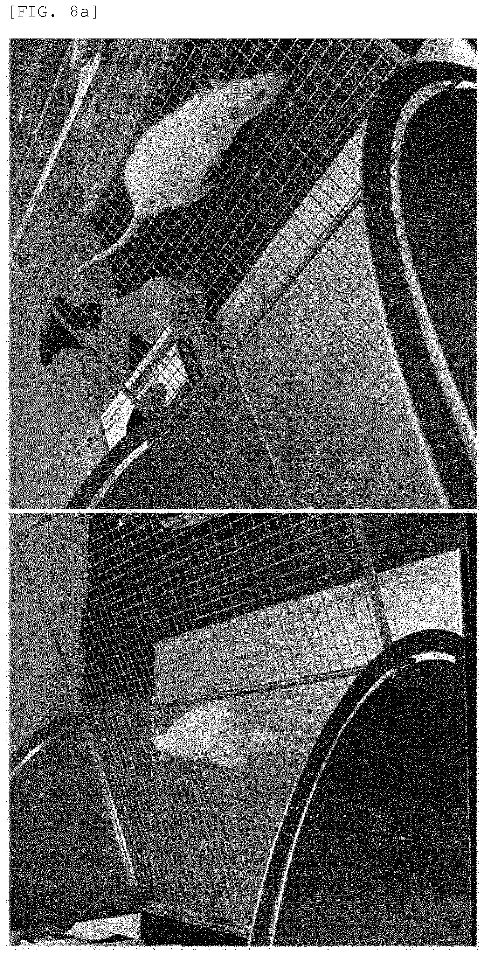

After ghMSC administration, inclined plane test was executed on day 1, 7, 14, 21 and 28. The date of administering ghMSC to the cerebral infarction sequela model was set as day 0, and then the sticky tape test was continued till day 1, 7, 14, 21 and 28 after administration. Specifically, a rat was placed on an inclined plane, followed by increasing an angle by 1° per second. The angle at losing balance was recorded, and the rat was fixed not to move during increase of the angle. At each direction, the measurement was repeated three times (FIG. 8a).

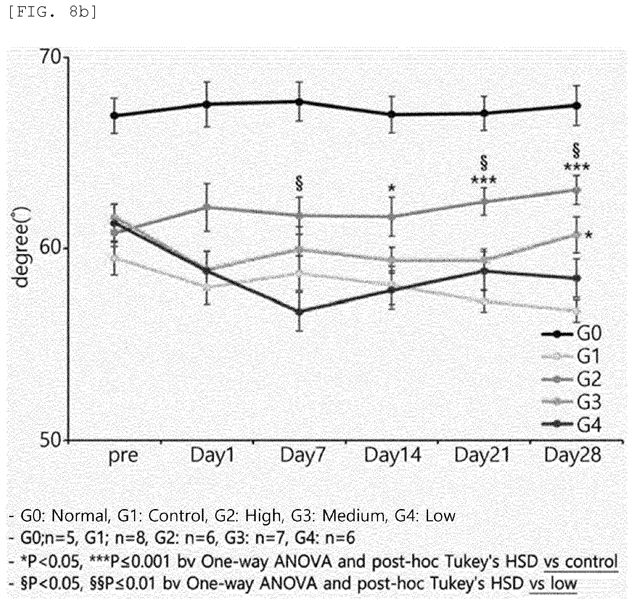

As a result, it was demonstrated in individual groups on 28 days after cell administration as follows: G0 : 66.1 ± 1.31, G1 : 56.75 ± 0.59, G2 : 63.06 ± 0.77, G3 : 60.71 ± 0.95, G4 : 58.44 ± 0.87, and it was confirmed that G2 group showed statistically significant difference from day 14 compared to G1 group. Further, G3 group also showed statistical significance on day 28 (P<0.05) (FIG. 8b). G0 group is a normal group and showed statistically significant difference in all times compared to G1 group (P<0.001).

**Example 2**

**1. Cell culture**

**(1) Culture of human mesenchymal stem cells (hMSCs)**

hMSC was prepared by the same method as in Example 1 except that passage culture was conducted 9 to 11 times

**(2) Induction of human mesenchymal stem cell into glia-like cell (ghMSC)**

ghMSC was prepared by the same method as in Example 1 except that hMSC of Example 2 was used.

**2. Comparison of treatment efficacy between hMSC before differentiation and ghMSC in cerebral infarction sequela model**

**(1) Configuration of experimental group and cell administration**

With reference to $2 \times 10^5$ cells amount with which treatment efficacies for cerebral infarction reduction and sequela improvement by ghMSC in the cerebral infarction sequela model were confirmed, ghMSC and hMSC were administered, respectively, and effects thereof were compared. Experimental procedures and schedules were applied in the same manner as described above. Further, a normal control group was added thus to establish the experimental groups as follows (Table 2).

[TABLE 2]

| Group | Total cell number | Amount |
|---|---|---|
| Normal | 0 | 0 μL |

(continued)

| Group | Total cell number | Amount |
|-------|-------------------|--------|
| hMSC | $2 \times 10^5$ cells | 15 μL |
| ghMSC | $2 \times 10^5$ cells | 15 μL |

**(2) MR image assay**

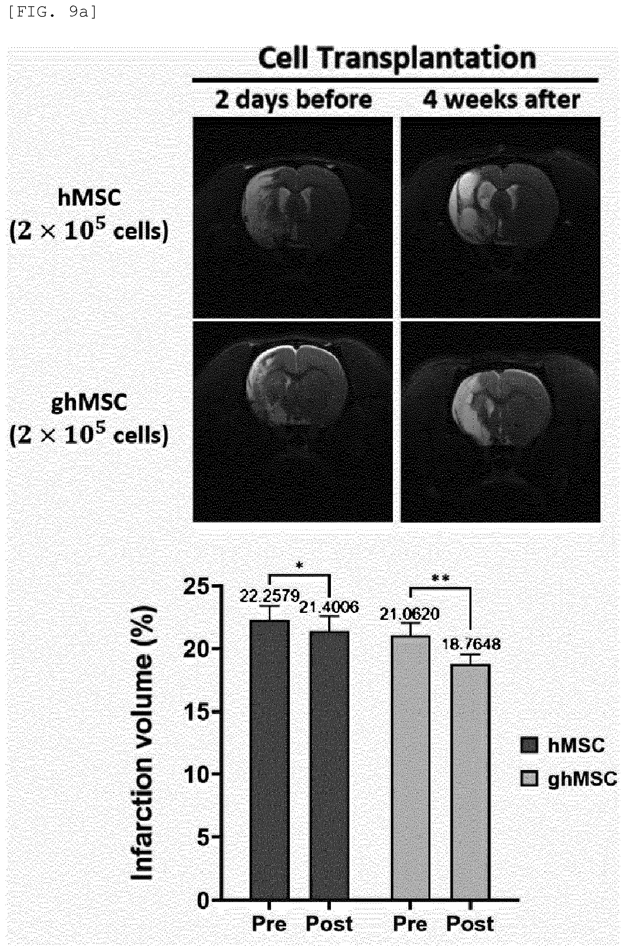

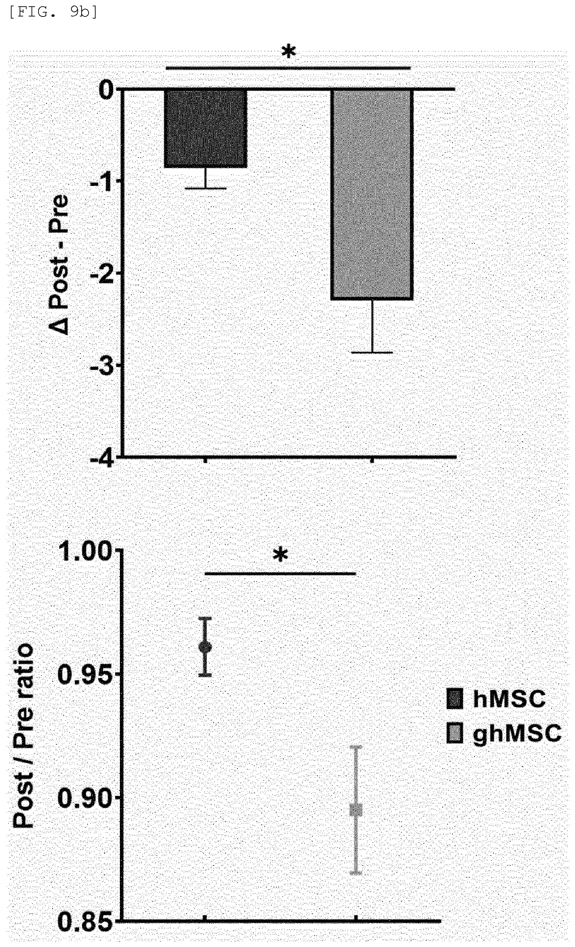

2 days before cell administration, a cerebral infarction volume was measured through MR image, followed by equally dividing the volume into groups. 28 days after cell administration, as a result of comparing a change in sizes of cerebral infarction, the hMSC administered group showed: $22.26 \pm 0.99$ before cell administration; and $21.401 \pm 1.03$ after cell administration. Further, the ghMSC administered group showed: $21.06 \pm 0.91$ before cell administration; and $18.76 \pm 0.72$ after cell administration. As a result of statistical analysis, when the values before and after cell administration were analyzed by Paired T-test, statistical significance was shown in the hMSC administered group ($p<0.05$). However, the changed value was larger in the ghMSC administered group, whereas P-value indicating statistical significance was smaller ($p<0.01$) (FIG. 9a). Further, volume changes were as follows: hMSC administered group: $-0.86 \pm 0.19$, ghMSC administered group: $-2.30 \pm 0.53$, demonstrating that the volume change was more greatly reduced in the ghMSC administered group and also confirming statistically significant difference ($p<0.05$). The ratio of the value after cell administration divided by the value before cell administration was confirmed as follows: hMSC administered group: $0.96 \pm 0.01$, ghMSC administered group: $0.90 \pm 0.02$, demonstrating that the ratio was greatly reduced in the ghMSC administered group, and also confirming statistically significant difference ($p<0.05$) (FIG. 9b)

**(3) Inclined plane test**

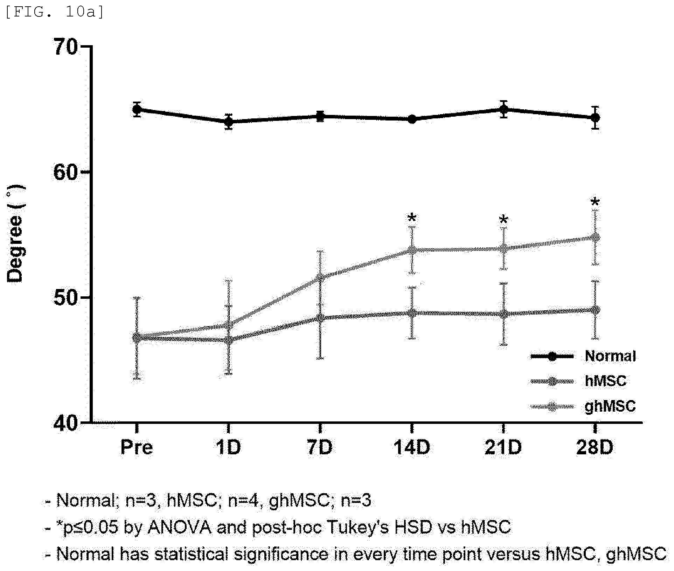

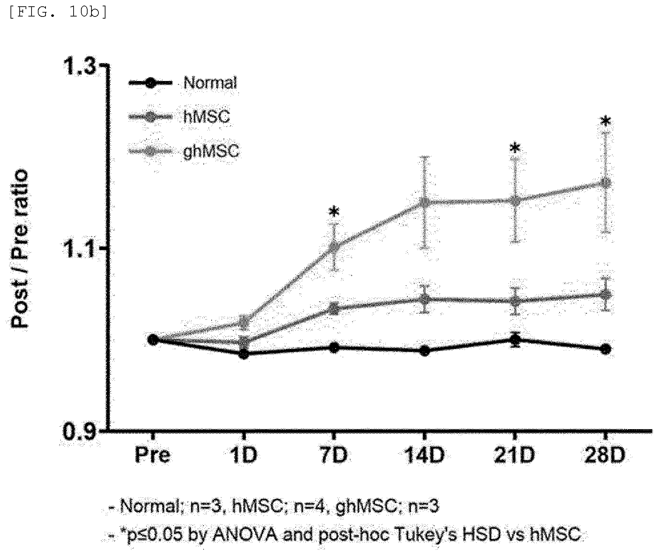

According to the inclined plane test that demonstrated a difference between the excipient control group and the ghMSC administered group in the previous experiments, it was intended to compare the degree of improvement of aftereffects between the above two groups. As a result, it was confirmed that, in individual groups on day 28 after cell administration: Normal: $64.33 \pm 0.42$, hMSC: $49 \pm 0.99$, ghMSC: $54.78 \pm 1.01$, and the ghMSC administered group showed increase in angle capable of holding on the inclined plane as compared to the hMSC administered group, and statistical significance appeared from day 14 after cell administration ($p<0.05$) (FIG. 10a). A normal control group showed statistically significant difference in all days compared to the hMSC administered group and the ghMSC administered group.

**3. Real time gene proliferation assay (real time qPCR)**

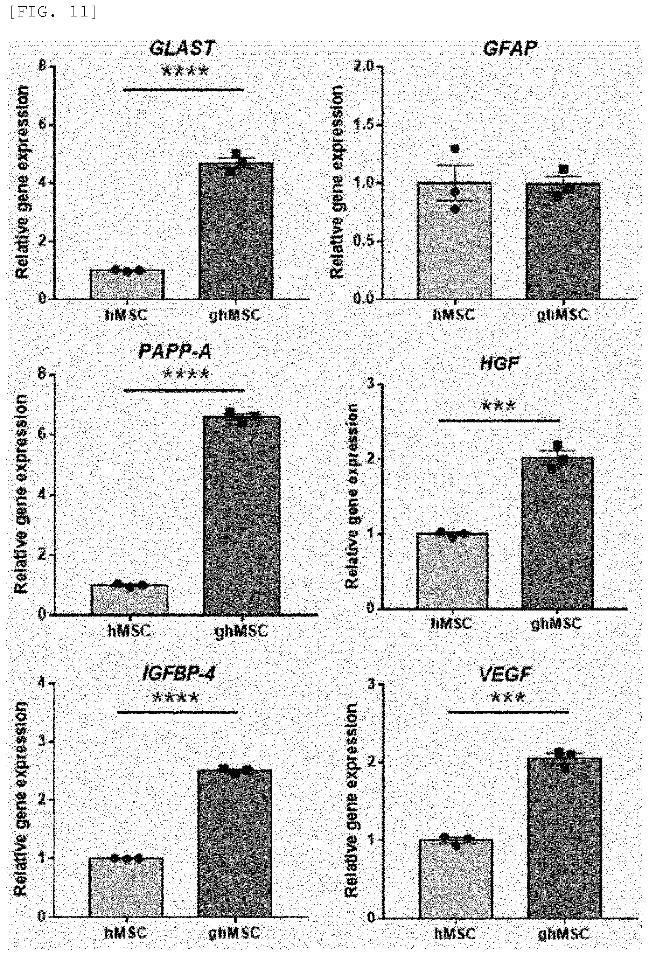

Total RNA separated according to the manual method using TriZol reagent was subjected to a reaction at 42 °C for 1 hour through M-MLV reverse transcriptase thus to synthesize cDNA. The synthesized cDNA was used as a template to compare gene expression through SYBR Green gene expression assays. A target gene was standardized by endogenous GAPDH, and comparison of a gene level was performed using a Ct value comparison method of the measured gene.
Referring to FIG. 11, it could be seen that the differentiated marker of ghMSC showed the tendency of significantly increased expression as compared to undifferentiated hMSC.

**4. Enzyme immunoassay (VEGF-A, IGFBP-4 ELISA)**

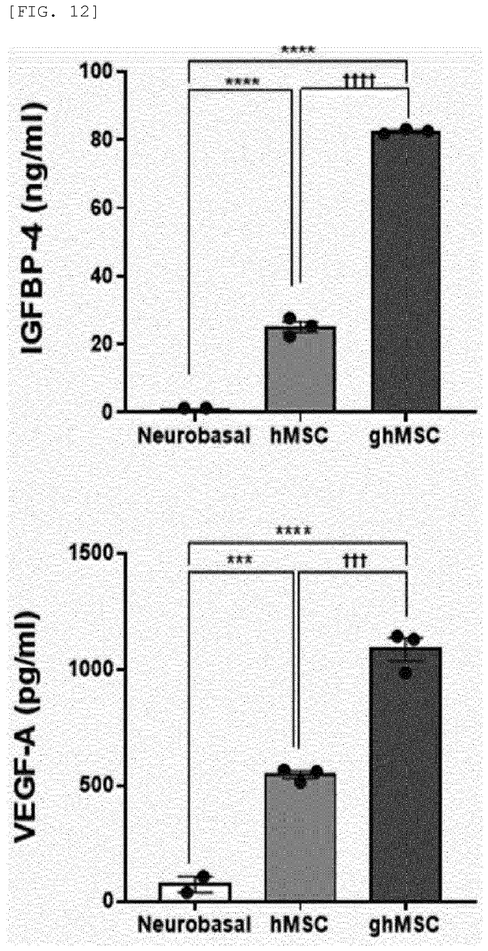

hMSC and ghMSC after completion of differentiation were cultured in a basal medium (F12) for 18 hours. According to RayBio® Human VEGF-A ELISA Kit (For Lysates), R&D Systems Co., DuoSet human IGFBP-4 ELISA kit manual method, a concentration of VEGF-A protein in hMSC-CM and ghMSC-CM was determined.
Referring to FIG. 12, it could be confirmed that, in both of VEGF and IGFBP-4, ghMSC-CM is expressed at a significantly high concentration, compared to hMSC-CM (One-way ANOVA Test by Tukey).
***$P < 0.001$,****$P < 0.0001$ versus Neurobasal; ††† $P < 0.001$, †††† $P< 0.0001$ versus hMS

**5. Flow cytometry**

After fixing the above cells using 4% formaldehyde for 20 minutes, the cells were washed twice with DPBS containing calcium ions and magnesium ions. For permeation of an antibody, Triton X-100 was diluted to 0.1% in PBS and the cells were treated with this solution for 1 hour, followed by washing twice with FACS buffer (5% FBS/PBS). After attaching fluorescence-adhered primary antibody to the cells, these were reacted at 4 °C for 1 hour. After washing the product twice with FACS buffer, it was subjected to assay in a flow cytometry device.

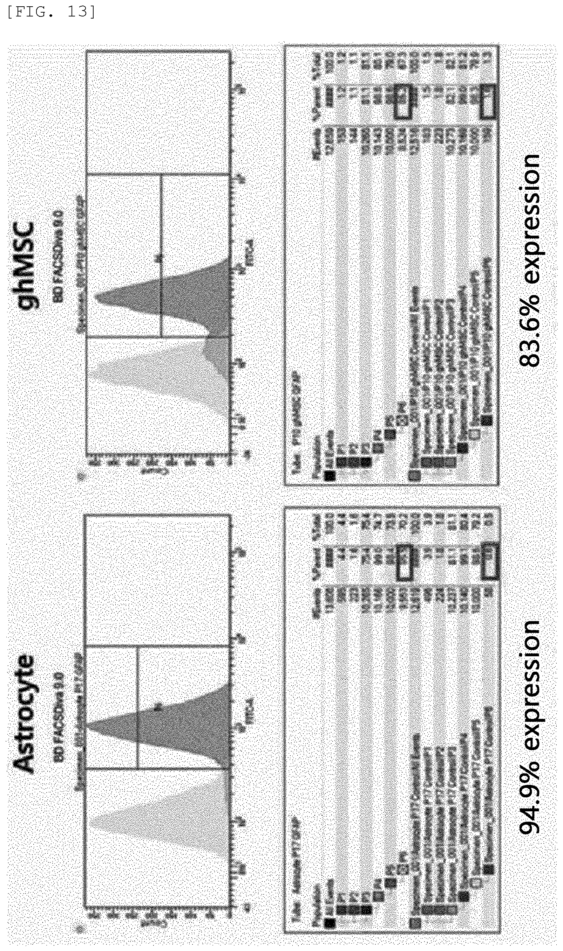

Referring to FIG. 13, when flow cytometry was performed in ghMSC, it could be seen that an amount of expression of GFAP was 83.6%, while astrocytes as a positive control group had 94.9% GFAP expression.

(ghMSC 83.6% GFAP expression, Astrocyte 94.9% GFAP expression)

### 6. Immunocytochemistry (ICC)

hMSC and ghMSC were fixed with 4% PFA in PBS, followed by permeabilization with 0.1% Triton X-100. As the primary antibody, mouse anti-GFAP (1:200), rabbit anti-S100 (1:250), GLAST-PE (1:50) were used for reaction overnight. Then, as the secondary antibody, Alexa 546 anti-rabbit IgG and Alexa 488 anti-mouse IgG were used, followed by staining the nucleus with 0.1 ug/ml DAPI. Thereafter, the product was observed through a reverse-phase fluorescence microscope.

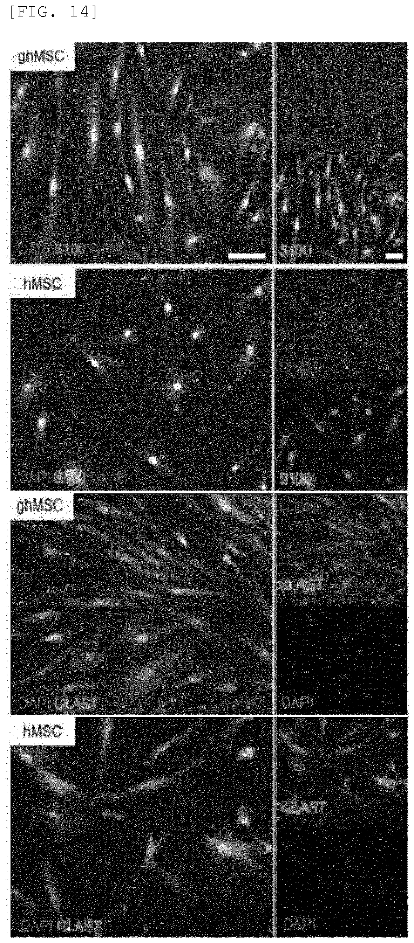

Referring to FIG. 14, it could be understood that astrocyte markers, that is, GFAP, S100 and GLAST protein expression are larger in ghMSC, compared to hMSC.

Astrocyte marker: GFAP (Glial fibrillary acidic protein), S100 (S100 Protein), GLAST (GLutamate Aspartate Transporter 1)

### 7. Cytokine expression assay (Cytokine array)

Undifferentiated hMSC and differentiation-terminated ghMSC were cultured for 18 hours in Neurobasal media. Then, according to RayBio® Human Cytokine Antibody Array C5 kit manual method, the tendency of cytokine expression in each of the neurobasal media, hMSC-CM and ghMSC-CM was investigated.

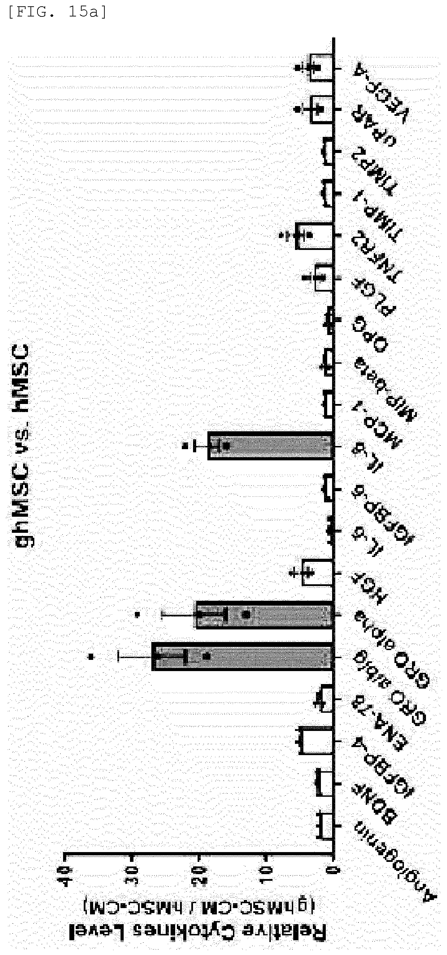

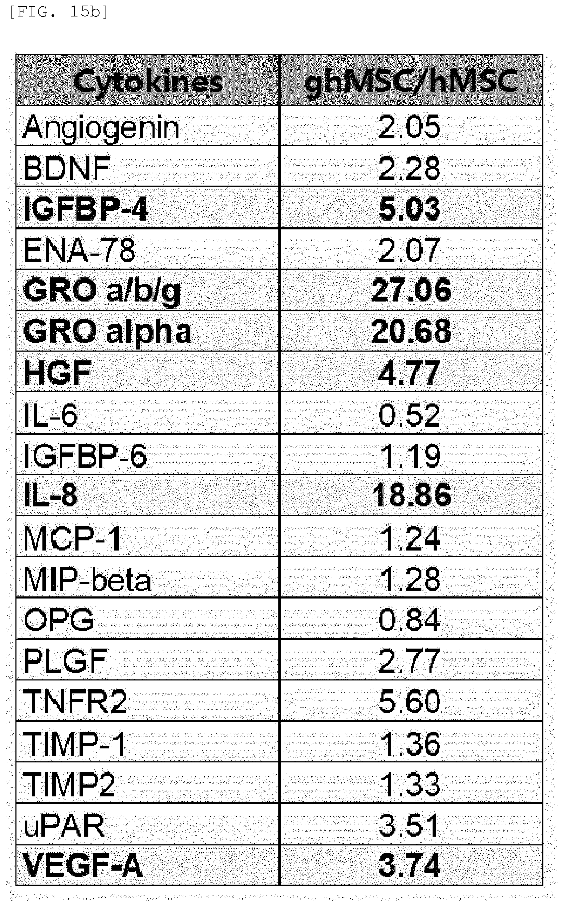

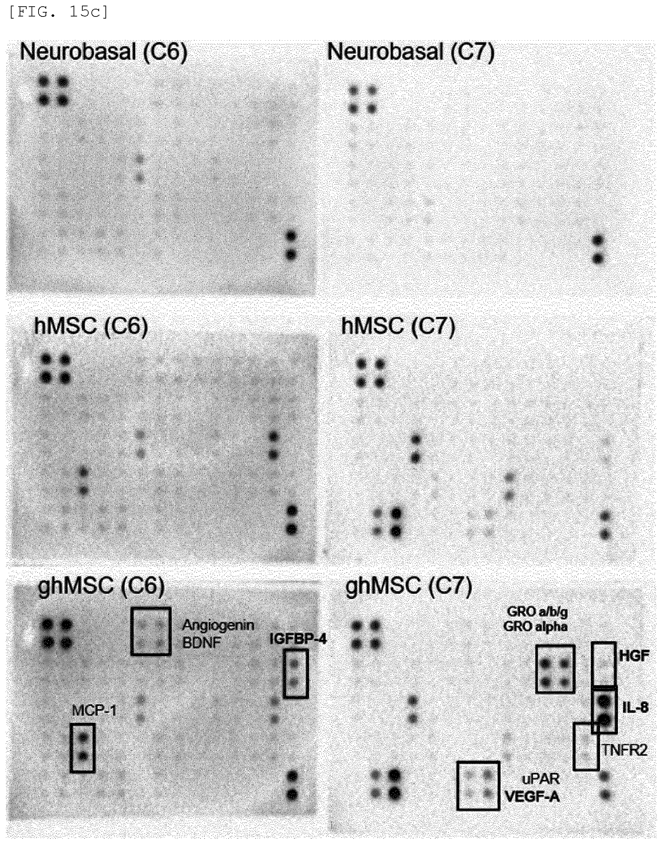

Referring to FIG. 15, it could be confirmed that cytokine expression of GRO a/b/g, GRO alpha (CXCL1), IL-8, IGFBP-4 and VEGF-A was increased in ghMSC-CM, as compared to the neurobasal (media) and hMSC-CM.

### 8. Ex vivo

### (1) Organotypic brain slice culture

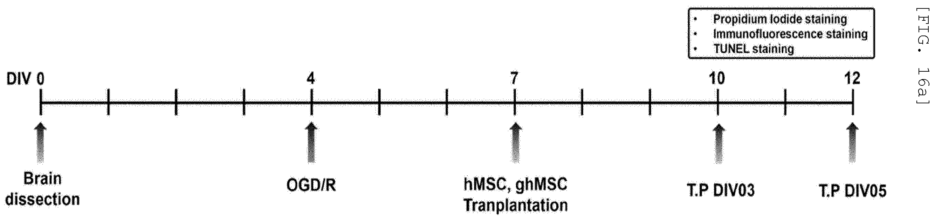

An experimental schematic view is shown in FIG. 16a.

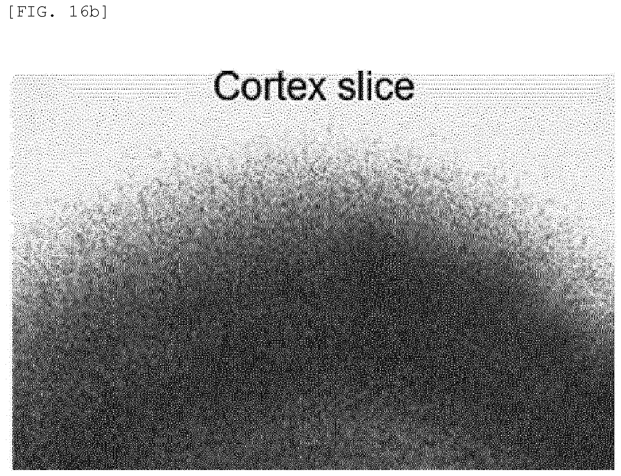

P7 (Postnatal 7, day 7 after birth) rat brain was extracted, and the brain was sliced with a constant thickness of 350 um by using a chopper (FIG. 16b)

The sliced tissue was cultured with a medium including 50% MEM + HEPES mixture, 25% Horse serum, 25% Hank's balanced salt solution (HBSS), 6.5 mg/mL Glucose and 1% Penicillin/Streptomycin,

After culturing for 4 days, the product was subjected to a reaction for 2 hours using 5 ug/mL of propidium iodide (PI) before oxygen-glucose deprivation (OGD), followed by observation using a fluorescence microscope and then measurement (F0).

To establish an ischemia-damaged model, the brain slice was treated with OGD solution, followed by bubbling 95% $N_2$ and 5% $CO_2$ gas to remove oxygen. Then, the slice was subjected to oxygen-glucose deprivation for 1 hour, followed by performing reoxygenation for 3 days, thereby establishing a chronic stroke model.

### (2) Propidium iodide (PI) uptake assay and image assay

After completing OGD/R for 72 hours, the product was treated with PI solution (5 $\mu$g/mL), followed by observing cell death evaluation through a fluorescence microscope (Ft).

For cell death evaluation, the medium was exchanged with PBS, the tissues were stored at 4 °C for 24 hours. Then, PI staining was performed, followed by observation through a fluorescence microscope (F fin).

Image assessment was performed by setting 8-bit through Image J, measuring a gray-value mean intensity, and then evaluating a percentage of cell death through Equation 2 below.

```
[Equation 1]
```

```
% Cell death evaluation formula = (Ft-F0)/(Ffin-F0) x 100
```

(F0 : Basal fluorescence intensity, Ft : OGD/Reoxygenation fluorescence intensity, Ffin : Cell all kills fluorescence intensity)

**(3) Assessment of cell death protection effects after transplantation of glia-like human mesenchymal stem cells (ghMSCs) and hMSC in OGD-induced brain slice stroke sequela model**

3 days after OGD/R performing, transplantation of ghMSC and hMSC in tissues was performed. Using a microcapillary tip, dissociation of ghMSC and hMSC was conducted, followed by counting the number of cells. Then, after spinning down again at 2000g for 3 minutes, transplantation in tissue was performed at $3 \times 10^4$ cells/1.5 ul/slice, and on day 5 and 7, cell death protection effects were evaluated.

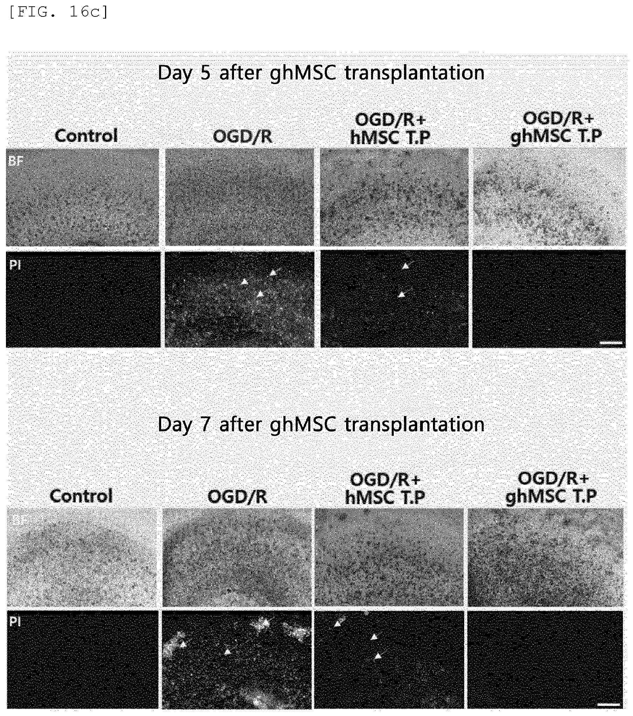

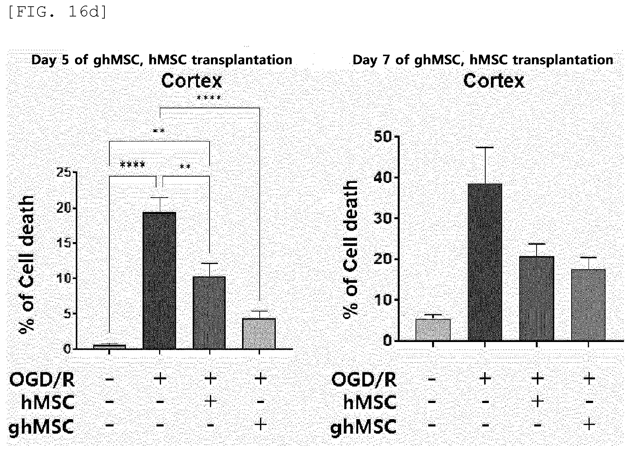

Referring to FIGS. 16c and d, OGD/R group in cortex showed significant increase in cell death rate as compared to the normal control group. Then, after transplantation of hMSC and ghMSC, it could be confirmed that the cell death rate in cortex was reduced on day 5. Further, although there was a tendency of reducing the cell death rate on day 7, statistical significance did not appear. Accordingly, it could be seen that cell death protection effects in the brain injury model was demonstrated 5 days after transplantation of ghMSC.

FIG. (A) illustrates the normal control group (Control), ischemic/reperfusion (OGD/R), hMSC transplantation (OGD/R+hMSC T.P.), and ghMSC transplantation (OGD/R+ghMSC T.P.) groups from the left. Further, the white arrow indicates cell death (Statistics: One way ANOVA test by Tukey).

**(4) Cell death assessment after transplantation of ghMSC and hMSC, and SB225002 (CXCR2 antagonist) treatment**

The experiment was executed in the same procedure as in the above item (3) except that pre-treatment using 40 ng/mL of CXCR2 antagonist (SB225002) was conducted for 3 days, thereby evaluating protection effects to cell death.

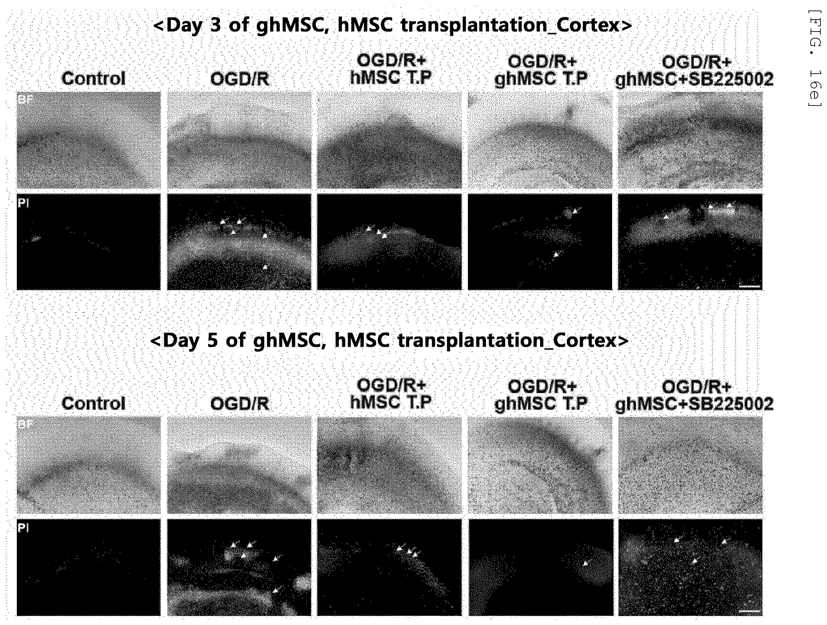

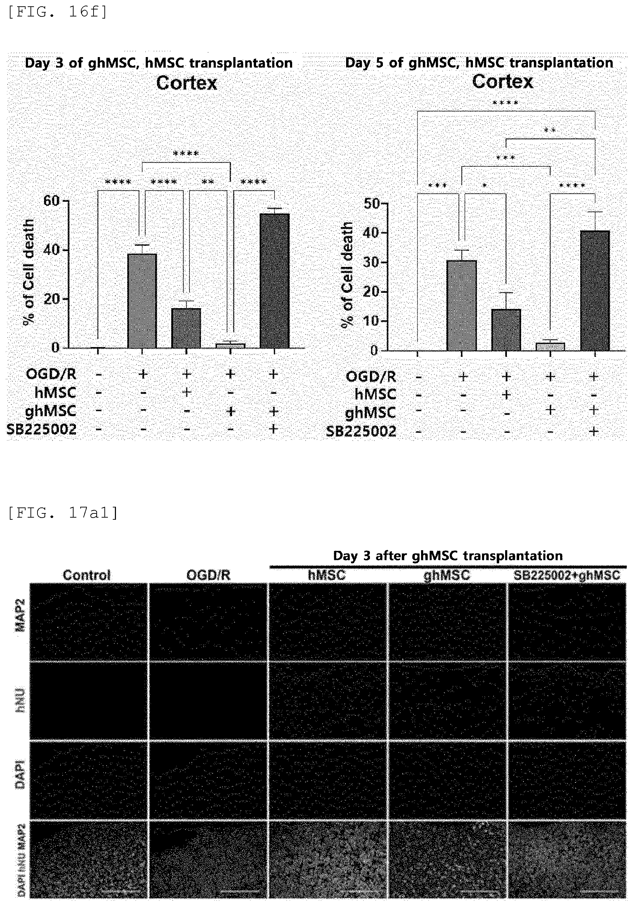

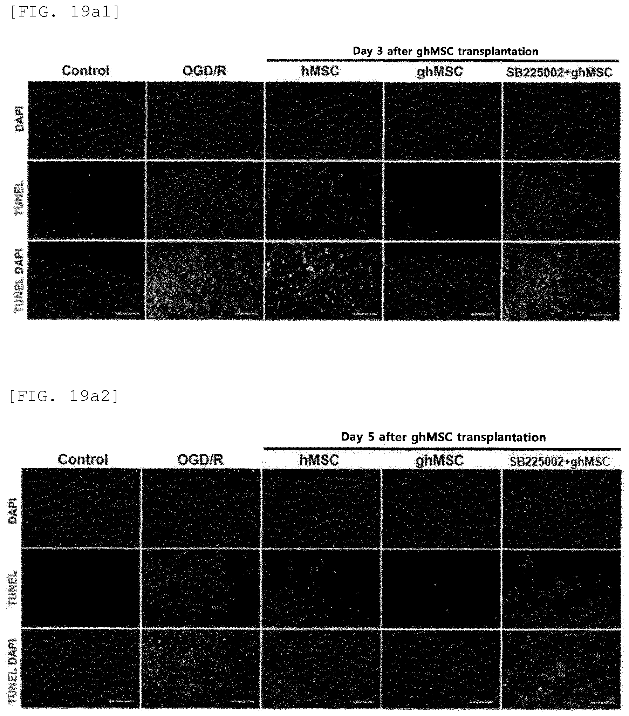

Referring to FIGS. 16e and f, and FIG. 19, both on day 3 and day 5, it could be seen that cell death was significantly reduced in the ghMSC group. Further, as a result of transplanting ghMSC after pre-treatment for 3 days using 40 ng/mL of CXCR2 antagonist (SB225002), it was demonstrated that the cell death rate was increased 3 days and 5 days after transplantation, which showed significant difference as compared to the ghMSC group.

**(5) Effect of ghMSC transplantation in tissue in *ex vivo* chronic stroke brain slice**

5 days after OGD/R performing, each of Control, OGD/R, OGD/R+hMSC and OGD/R+ghMSC tissues was washed with PBS, fixed with 4% PFA. On the next day, it was washed three times by 10 minutes a time.

After permeabilization with 0.5% Triton X-100 (1% BSA in PBS-T) for 20 minutes, blocking was performed with 3% BSA (0.1% TX-100) while gently stirring for 1 hour.

A primary antibody MAP2 (Rabbit, 1:400), human Nucleus (Mouse, 1:200) was bound to the tissue at 4 °C. On the next day, the treated product was washed with PBS, then secondary antibodies Alexa 488 rabbit, 1:200 and Alexa 546 mouse 1:200 were bound for 1 hour, followed by reacting the product with 0.1 μg/mL DAPI while shielding light. After washing with PBS, the product was sealed on a slide and observed using LEICA fluorescence microscope.

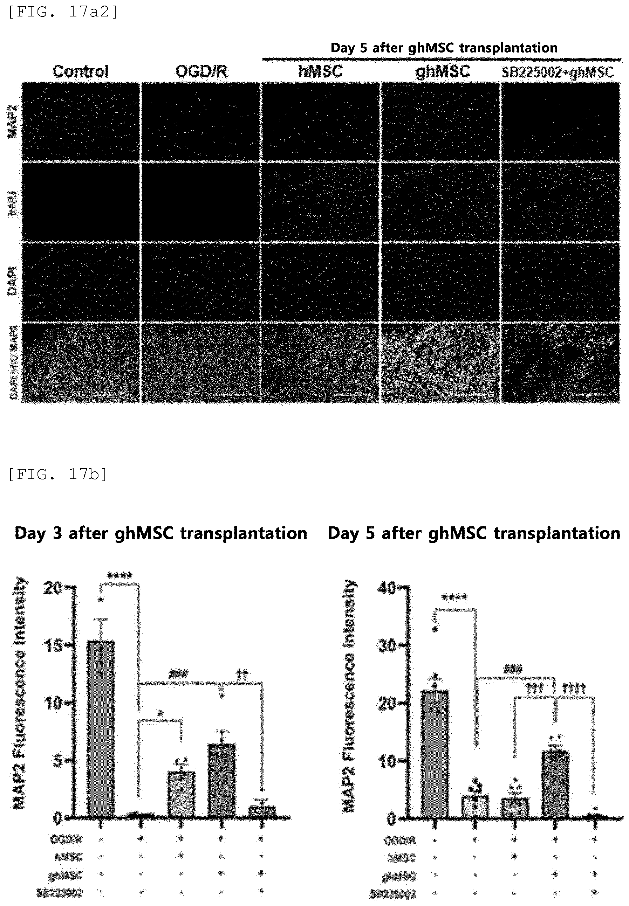

Referring to FIGS. 17a and b, 3 days and 5 days after ghMSC transplantation, it was shown that MAP2 fluorescent intensity was significantly increased in a brain tissue section of cortex, as compared to OGD/R group and a control group (without cell transplantation). Further, 7 days after transplantation of hMSC and ghMSC, a tendency for the fluorescence intensity of MAP2 of cortex to increase could be confirmed. Therefore, it could be understood that, 3 days after transplantation of ghMSC in the cerebral infarction sequela model, the brain injury model showed cell death protection effects and increase of neuronal marker, while having improvement in cell survival and neural regeneration on day 5.

**(6) TUNEL Assay**

Terminal deoxynucleotidyl transferase dUTP nick-end labeling (TUNEL) staining was executed by means of in situ Apoptosis Detection Kit (TAKARA) according to a protocol of the manufacturer for detection of fragmented DNA, which was simply treated with a permeable buffer for 10 minutes. Then, a mixture of TdT Enzyme and a labeling safe buffer in 1:9 was dispensed on the brain slice, followed by reacting the same in an incubator for 90 minutes. After washing, nucleus staining was performed with 0.1 μg/mL of DAPI. The product was sealed on a slide and observed using LEICA inverted fluorescence microscope.

Referring to FIGS. 19a and b, 3 days and 5 days after ghMSC transplantation to a brain tissue section, it could be seen that TUNEL fluorescent intensity was reduced, and cell death was significantly reduced in hMSC and ghMSC transplanted groups. However, when ghMSC was transplanted to the brain tissue section treated with CXCR antagonist, it could be confirmed that cell death was increased without such cell protection effects. Therefore, it could be understood that CXCR2 activity participated in cell protection effects 3 days and 5 days after ghMSC transplantation.

### 9. In vitro

#### (1) ReNReNcell VM cell culture

ReNReNcell VM cell is an immortalized human neural stem cell line, and this was cultured with a culture medium which is a mixed medium of DMEM/F-12, 1 % N2, 1 % B27, 20 ng/mL basis fibroblast growth factor, 2 ng/mL epidermal growth factor, 1% penicillin/streptomycin and 1% antimycotic/antibiotics. Further, passage culture was performed once every three days.

#### (2) Establishment of OGD-induced ReNReNcell VM cell stroke sequela model and CXCR2 antagonist treatment

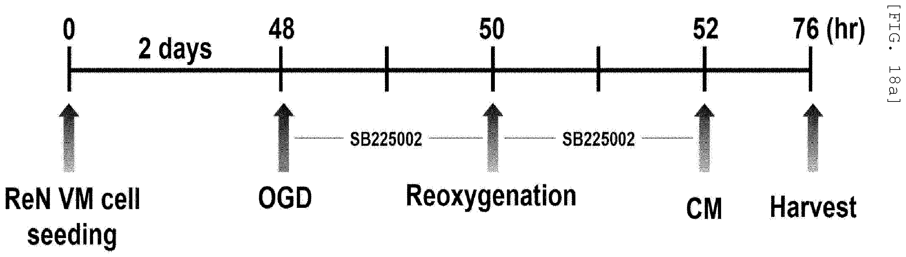

The experimental schematic view is shown in FIG. 18a.

After culturing ReNReNcell VM cells for 2 days, the cells were washed twice with PBS and subjected to oxygen-glucose deprivation (OGD) to induce ischemia. After removing oxygen from the glucose-removed DMEM medium through bubbling using a mixed gas of 95% $N_2$ and 5% $CO_2$, the ReNReNcell VM cells were cultured in a sealed chamber with injection of low oxygenic gas mixture (95% $N_2$, 5% $CO_2$) at 37 °C and exposed for 2 hours. On the other hand, the CXCR2 antagonist + ghMSC-CM group was treated with CXCR2 antagonist for 2 hours from OGD condition. Thereafter, in order to establish a chronic stroke model, the treated group was washed with a normal culture medium (DMEM/F12, 1% N2 1%, B27), followed by dispensing the normal culture medium thereto and culturing for 2 hours. Otherwise, reoxygenation was performed with a medium containing 400 ng/mL CXCR2 antagonist in a 5% $CO_2$ incubator for 2 hours. OGD/R-induced cells were treated with Neurobasal media for 24 hours and then with ghMSC-CM for 24 hours, followed by confirming protein expression.

#### (3) Western blotting

After washing the cells with cold PBS twice, protein was separated in RIPA buffer (50 mM Tris-HCl, pH 7.4, 1% Triton X-100, 0.25% sodium deoxycholate, 150 mM NaCl, 1 mM $Na_3VO_4$, 0.2 mg/ml leupeptin, 0.2 mg/ml aprotinin, 0.1 M PMSF, and 0.5 M sodium fluoride) containing protease and phosphatase inhibitors. After lysate centrifugation, a protein concentration was quantified using Lowry protein analysis method, by dispensing 5 × SDS buffer. Then, after boiling at 100 °C for 5 minutes, the protein was cooled on ice and a predetermined amount (10 μg) of protein was separated through 12% sodium dodecyl sulfate-polyacrylamide gel electrophoresis (SDS-PAGE) at 120 V for 2 hours, and then transferred to a polyvinylidene difluoride (PVDF) membrane for 90 minutes.

Referring to the experimental schematic view of FIG. 18a, in order to confirm signal transfer through CXCR2 as an upper receptor of pAkt, the product was treated with 400 ng/ml of CXCR2 antagonist, followed by performing ghMSC-CM for 24 hours and confirming through protein analysis.

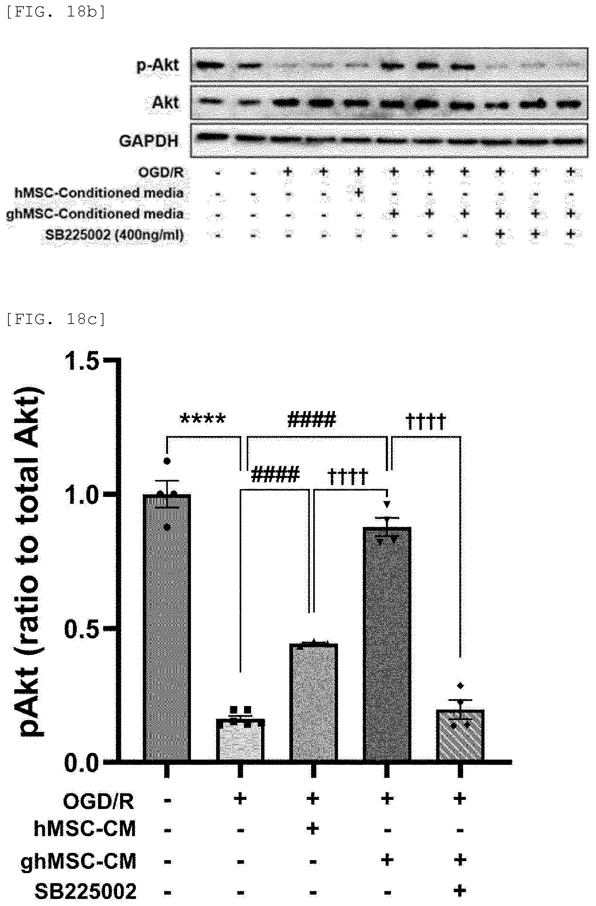

As a result, it could be confirmed that pAkt was significantly reduced in OGD/R group, as compared to the control group (One way ANOVA test by Tukey, **** P < 0.0001 vs Control). Further, the ghMSC-CM group showed statistically significant increase of pAkt expression, as compared to the OGD/R group and hMSC-CM group (****(#### P < 0.0001 vs hMSC-CM, ****#### P< 0.0001 vs ghMSC-CM). Further, as compared the ghMSC-CM group, it could be seen that pAkt expression level was statistically and significantly reduced in the CXCR2 antagonist + ghMSC-CM group (****(†††† P < 0.0001 vs ghMSC-CM). Therefore, it could be confirmed that ghMSC-CM increased cell survival signal transfer of ReNReNcell VM through CXCR2 (FIGS. 18b and c) .

### 10. Molecular biological assessment using ghMSC transplanted brain tissues in cerebral infarction sequela animal model

#### (1) Immunohistochemistry (IHC)

Immunohistochemistry (IHC) using brain tissues was executed by the following method. Specifically, the tissue used in IHC experiment was cultured in 4% Paraformaldehyde for 3 days and fixed. Then, in order to prevent damage to the tissue due to ice crystals in the frozen section, the medium was replaced with 30% sucrose, followed by storage for 2 days or more. After the tissue around an administration site was attached in a thickness of 10 um to a slide, it was cultured with 0.3% $H_2O_2$ for 10 minutes, and non-specific protein was blocked with a solution including 1% BSA dissolved in TBS containing 1% FBS. After culturing a purposed antibody at 4 °C overnight, on the next day, the secondary antibody was attached, followed by mounting the same on a cover slide using a mounting solution including DAPI. After drying for about 24 hours, signals appearing by the band of wavelength through LEICA microscope (DMI 4000) were observed.

**(2) Western blotting**

Western blotting using the brain tissues was executed by the following method. The brain tissues used for WB were rapidly cooled in liquid nitrogen instantly after autopsy, followed by storage in a deep freezer at -80 °C. In the experiment, the hemisphere of the brain having cerebral infarction was used. For the normal group, tissues at the same side were used. The tissues were homogenized in RIPA II cell lysis solution including 100 mM sodium orthovanadate, 100 mM phenylmethylsulfonyl fluoride, 100mM sodium fluoride and 0.5% protease inhibitor cocktail by means of Q55 sonicator (Qsonica). The proteins obtained by centrifugation were quantified through BCA Assay and used in the experiments with equal amount of 30 $\mu$g. The protein admixed with 4x sample buffer was cultured at 70 °C for 10 minutes, cooled on ice, and then introduced to 4-12% gradient gel and separated by size at 200 V for 45 minutes, followed by transferring the same to PVDF membrane at 250 mA for 2 hours. After blocking the membrane in 2% skim milk, this membrane was cultured along with an antibody at 4 °C overnight. On the next day, after the secondary antibody culture, an expression amount was measured using chemidoc through ECL solution.

**(3) Confirmation of cell residual in tissue using human specific antibody**

To confirm whether the administered ghMSC is remained in the tissue, IHC was performed using a specific antibody bound to only the nucleus of a human body (human nuclei, MAB1281, 1:200). As a result, it could be confirmed that ghMSC exists around the administration site till 4 weeks after cell administration. Further, it was also confirmed that there is no expression in the neurobasal media-administered control group (FIG. 20a).

**(4) Confirmation of cell proliferation through 5-bromo-2'-deoxyuridine (BrdU)**

In order to detect cells proliferated in the body, 50 mg/kg of BrdU (Sigma-Aldrich) was intraperitoneally injected three times with an interval of 12 hours before animal autopsy. As a result of investigating the condition with BrdU antibody (B2531, 1:200), it could be seen that the ghMSC administered group was highly expressed at the administration site, compared to the excipient control group, and was not inter-positioned with ghMSC stained with PHK 26. Further, the difference in BrdU expression was more extremely exhibited around the cell administration site, and it could be confirmed that a large number of cells is proliferated in the ghMSC administered group (FIG. 20b).

**(5) Confirmation of phospho-Akt and neuron expression related to cell survival**

Using the antibody (9271S, 1:100) of phosphor-Akt protein known to play a significant role in cell survival control and the antibody bound to the nucleus of neuron, that is, NeuN (MAB377, 1:500), the degrees of expression were compared through IHC and Western blotting. As a result of IHC, the number of neurons at the hippocampal CA3 portion around the administration site and pAkt expression were higher in the ghMSC administered group, compare to the control group. These results are also confirmed through Western blotting. Specifically, as a result of GAPDH quantification, the condition similar to IHC could be confirmed. After repeated experiments three times, the results were divided by the value of the normal group, and then illustrated in graphs. Both of pAkt and NeuN in the ghMSC administered group showed statistically and significantly higher expression levels, compared to the control group (*p<0.05) (FIG. 20c)

**(6) CD31 for confirmation of neovascularization**

To investigate blood vessels newly generated in the brain, an antibody adhered to vascular endothelial cells, that is, CD31 (ab9498, 1:100) was used. All of the three groups were observed for cortex around the administration site. As a result of IHC, the excipient control group showed considerably low expression amount, whereas the ghMSC administered group was expressed similar to the normal group. As a result of confirming also these groups through Western blotting, the same condition as shown in IHC was exhibited. As compared to the control group, the ghMSC administered group also showed statistically significant difference (*p<0.05) (FIG. 20d).

**(7) Confirmation of PSD-95 exhibiting synapse plasticity**

Postsynaptic density protein (PSD) 95 is a protein closely relevant to synapse plasticity. Using this antibody (ab18258, 1:100), the expression was compared through IHC and Western blotting. As a result of IHC, PSD-95 molecules were largely expressed in the internal capsule around the administration site, and it could be confirmed that the ghMSC administered group showed higher expression amount, compared to the control group. Further, these results were also confirmed through Western blotting and, as a result of GAPDH quantification, the same condition as in IHC was exhibited. Further, when the result was divided by the value of the normal group after repeated experiments three times, it could be seen that the expression in the ghMSC administered group was statistically and significantly higher, compared to the control group (**P<0.01) (FIG. 20e).

**(8) Confirmation of CXCR2 for identifying functional mechanism of ghMSC**

As described above, it was confirmed that, when CXCR2 was blocked, effects of ghMSC are deteriorated through *in vitro* and ex *vivo* studies. Further, it was also demonstrated that neuron expression and cell survival due to the above results were also reduced. On the above ground, it was intended to compare CXCR2 expression *in vivo* between the groups. First, as a result of IHC, it could be confirmed that CXCR2 expression in the ghMSC administered group is higher than the control group in all cortex in the administration site and around the same. On the other hand, for the normal group, the portion at the same coordinate as the administration site was observed. Further, since it was not inter-positioned with the PKH26 stained ghMSC, it could be expected that CXCR2 expression was increased in the cell of the rat itself. The expression was also compared through Western blotting and showed the condition similar to the result of IHC. Through repeated experiments three times, statistically significant difference could be seen in the ghMSC administered group compared to the control group (*p<0.05) (FIG. 20f).

**Claims**

1. A pharmaceutical composition for alleviation or treatment of cerebral infarction sequela, comprising glia-like cells differentiated from human mesenchymal stem cells (hMSCs).

2. The pharmaceutical composition according to claim 1, wherein the glia-like cell expresses one or more marker genes selected from the group consisting of GFAP, GLAST, HGF, VEGF, IGFBP-4 and PAPP-A.

3. The pharmaceutical composition according to claim 1, wherein the human mesenchymal stem cell is derived from tissues selected from the group including bone marrow, fat, peripheral blood, cord blood, muscle and skin.

4. The pharmaceutical composition according to claim 1, wherein the human mesenchymal stem cell is passage-cultured twice to 15 times.

5. The pharmaceutical composition according to claim 1, wherein the cerebral infarction sequela is one or more selected from the group including paralysis, dysphagia, cognitive impairment, aphasia and stuttering.

[FIG. 1a]

[FIG. 1b]

[FIG. 1c]

[FIG. 1d]

| Markers | Percentage |
|---|---|
| S100+ | 44.89 ± 4.12 |
| GFAP+ | 40.41 ± 4.45 |
| S100+/GFAP+ | 40.41 ± 4.45 |

[FIG. 2]

[FIG. 3]

EP 4 356 916 A1

Figure 17

Interaural 9.20 mm

Bregma 0.20 mm

[FIG. 4]

[FIG. 5a]

Whole brain area measurement

[FIG. 5b]

[FIG. 6a]

- G1: Control, G2: High, G3: Medium, G4: Low
- G1; n=8, G2: n=6, G3: n=7, G4: n=6
- *$P \leq 0.05$ by One-way ANOVA and post-hoc Tukey's HSD vs control

[FIG. 6b]

- G1: Control, G2: High, G3: Medium, G4: Low
- G1; n=8, G2: n=6, G3: n=7, G4: n=6
- *P≤0.05 by One-way ANOVA and post-hoc Tukey's HSD vs control

[FIG. 7a]

[FIG. 7b]

[FIG. 7c]

- G0: Normal, G1: Control, G2: High, G3: Medium, G4: Low
- G0;n=5, G1; n=8, G2: n=6, G3: n=7, G4: n=6
- Analysis by One-way ANOVA

[FIG. 8a]

[FIG. 8b]

- G0: Normal, G1: Control, G2: High, G3: Medium, G4: Low
- G0;n=5, G1; n=8, G2: n=6, G3: n=7, G4: n=6
- *P<0.05, ***P≤0.001 bv One-way ANOVA and post-hoc Tukey's HSD vs control
- §P<0.05, §§P≤0.01 bv One-way ANOVA and post-hoc Tukey's HSD vs low

[FIG. 9a]

[FIG. 9b]

[FIG. 10a]

- Normal; n=3, hMSC; n=4, ghMSC; n=3
- *p≤0.05 by ANOVA and post-hoc Tukey's HSD vs hMSC
- Normal has statistical significance in every time point versus hMSC, ghMSC

[FIG. 10b]

- Normal; n=3, hMSC; n=4, ghMSC; n=3
- *p≤0.05 by ANOVA and post-hoc Tukey's HSD vs hMSC

[FIG. 11]

[FIG. 12]

[FIG. 13]

[FIG. 14]

[FIG. 15a]

[FIG. 15b]

| Cytokines | ghMSC/hMSC |
|---|---|
| Angiogenin | 2.05 |
| BDNF | 2.28 |
| **IGFBP-4** | **5.03** |
| ENA-78 | 2.07 |
| **GRO a/b/g** | **27.06** |
| **GRO alpha** | **20.68** |
| **HGF** | **4.77** |
| IL-6 | 0.52 |
| IGFBP-6 | 1.19 |
| **IL-8** | **18.86** |
| MCP-1 | 1.24 |
| MIP-beta | 1.28 |
| OPG | 0.84 |
| PLGF | 2.77 |
| TNFR2 | 5.60 |
| TIMP-1 | 1.36 |
| TIMP2 | 1.33 |
| uPAR | 3.51 |
| **VEGF-A** | **3.74** |

[FIG. 15c]

[FIG. 16a]

- Propidium Iodide staining
- Immunofluorescence staining
- TUNEL staining

DIV 0        4        7        10        12

Brain
dissection

OGD/R

hMSC, ghMSC
Tranplantation

T.P DIV03

T.P DIV05

[FIG. 16b]

Cortex slice

[FIG. 16c]

Day 5 after ghMSC transplantation

Day 7 after ghMSC transplantation

[FIG. 16d]

[FIG. 16e]

[FIG. 16f]

**Day 3 of ghMSC, hMSC transplantation**
**Cortex**

**Day 5 of ghMSC, hMSC transplantation**
**Cortex**

[FIG. 17a1]

Day 3 after ghMSC transplantation

Control | OGD/R | hMSC | ghMSC | SB225002+ghMSC

[FIG. 17a2]

[FIG. 17b]

[FIG. 18a]

EP 4 356 916 A1

[FIG. 18b]

[FIG. 18c]

[FIG. 19a1]

**Day 3 after ghMSC transplantation**

[FIG. 19a2]

**Day 5 after ghMSC transplantation**

[FIG. 19b]

Day 3 after ghMSC transplantation

Day 5 after ghMSC transplantation

[FIG. 20a]

[FIG. 20b]

**Injection site**

**Peri-infarct site**

[FIG. 20c1]

[FIG. 20c2]

[FIG. 20d1]

[FIG. 20d2]

[FIG. 20e1]

[FIG. 20e2]

**PSD-95/GAPDH**

[FIG. 20f1]

[FIG. 20f2]

## INTERNATIONAL SEARCH REPORT

International application No.

**PCT/KR2023/006017**

### A. CLASSIFICATION OF SUBJECT MATTER

**A61K 35/28**(2006.01)i; **A61K 35/30**(2006.01)i; **A61P 25/00**(2006.01)i; **A61P 9/10**(2006.01)i; **C12N 5/079**(2010.01)i; **A61P 9/00**(2006.01)i

According to International Patent Classification (IPC) or to both national classification and IPC

### B. FIELDS SEARCHED

Minimum documentation searched (classification system followed by classification symbols)

A61K 35/28(2006.01); A61K 35/30(2006.01); C12N 5/079(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 유사교세포(glia-like cell), 인간 중간엽 줄기세포(human mesenchymal stem cell), 뇌경색(cerebral infarction), 후유증(aftereffect), 치료(treatment)

### C. DOCUMENTS CONSIDERED TO BE RELEVANT

| Category* | Citation of document, with indication, where appropriate, of the relevant passages | Relevant to claim No. |
|---|---|---|
| Y | KR 10-2021-0023781 A (SEOUL NATIONAL UNIVERSITY R&DB FOUNDATION et al.) 04 March 2021 (2021-03-04)<br>See paragraphs [0010], [0013], [0021], [0051], [0053], [0061] and [0078]. | 1-5 |
| Y | LLORENTE, Irene L. et al. Patient-derived glial enriched progenitors repair functional deficits due to white matter stroke and vascular dementia in rodents. Science Translational Medicine. 2021, vol. 13, eaaz6747, pp. 1-17.<br>See abstract; and pages 1, 4 and 12. | 1-5 |
| Y | SON, Jeong-Woo et al. Glia-like cells from late-passage human MSCs protect against ischemic stroke through IGFBP-4. Molecular Neurobiology. 2019, vol. 56, no. 11, pp. 7617-7630 (inner pp. 1-14).<br>See abstract; and pages 2, 8, 10 and 13. | 1-5 |
| A | STANCIOIU, Felician et al. CD271+ stem cell treatment of patients with chronic stroke. Experimental and Therapeutic Medicine. 2020, vol. 20, pp. 2055-2062.<br>See entire document. | 1-5 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **07 August 2023** | **07 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2023/006017** |

**C.      DOCUMENTS CONSIDERED TO BE RELEVANT**

| Category* | Citation of document, with indication, where appropriate, of the relevant passages | Relevant to claim No. |
|---|---|---|
| A | KR 10-2011-0104684 A (SEOUL NATIONAL UNIVERSITY R&DB FOUNDATION) 23 September 2011 (2011-09-23)<br>        See entire document. | 1-5 |

**INTERNATIONAL SEARCH REPORT**

| International application No. |
|---|
| **PCT/KR2023/006017** |

---

Box No. I          Nucleotide and/or amino acid sequence(s) (Continuation of item 1.c of the first sheet)

---

1. With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

   ☐  accompanied by a statement to the effect that the sequence listing does not go beyond the disclosure in the international application as filed.

2. ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/KR2023/006017**

| Patent document cited in search report | | | Publication date (day/month/year) | Patent family member(s) | | | Publication date (day/month/year) |
|---|---|---|---|---|---|---|---|
| KR | 10-2021-0023781 | A | 04 March 2021 | KR | 10-2503876 | B1 | 27 February 2023 |
| | | | | US | 2022-0331368 | A1 | 20 October 2022 |
| | | | | WO | 2021-034162 | A1 | 25 February 2021 |
| KR | 10-2011-0104684 | A | 23 September 2011 | KR | 10-1147412 | B1 | 22 May 2012 |

Form PCT/ISA/210 (patent family annex) (July 2022)